# DEMANDE DE BREVET EUROPEEN

(11) **EP 4 674 343 A1**
(43) Date de publication de la demande: **07.01.2026**
(21) Numéro de dépôt: 25186009.4
(22) Date de dépôt: 27.06.2025
(51) Int. Cl.: A61B 5/0537, A61B 5/00

(54) **MÉTHODE ET DISPOSITIF DE MESURE DE COMPOSITION CORPORELLE AVEC DÉTECTION D'ERREUR**

(30) Priorité: 01.07.2024 FR 2407163
(71) Demandeur: Withings, 92130 Issy-Les-Moulineaux (FR)
(72) Inventeur: Pons, Arthur, 92130 Issy-les-Moulineaux (FR); Léger, Charlotte, 92130 Issy-les-Moulineaux (FR)
(74) Mandataire: Withings IP

(57) **Abrégé**

La présente description concerne un dispositif de mesure (100) de composition corporelle configuré pour : effectuer des mesures d'impédance sur un utilisateur (U); comparer l'impédance mesurée à une donnée de référence générer un signal d'erreur sur la base de la comparaison.

## Description

La présente invention concerne un dispositif de mesure. En particulier, le dispositif de mesure est configuré pour mesurer au moins une impédance d'une portion du corps d'un utilisateur et de déterminer au moins une donnée relative à la composition corporelle de l'utilisateur.

### Etat de la technique

Parmi les dispositifs de mesure d'impédancemétrie, les pèse-personnes impédancemétriques qui permettent une mesure de poids et une mesure d'impédance de l'utilisateur sont les plus répandues. Ces pèse-personnes impédancemétriques fonctionnent en envoyant un courant électrique faible à travers au moins une partie du corps de l'utilisateur en train de se peser. Ce courant traverse les tissus avec des propriétés de résistances différentes (appelée impédance), la graisse résistant par exemple plus que les tissus musculaires, ce qui permet de déterminer une composition corporelle de l'utilisateur.

On peut citer deux types principaux de pèse-personnes impédancemétriques, à savoir les pèse-personnes dite "classique" comprenant uniquement une base apte à recevoir les pieds de l'utilisateur et les pèse-personnes dite "avec poignée" comportant en outre de la base une poignée apte à être saisie par l'utilisateur lors de la pesée. Alors qu'un pèse-personne impédancemétrique classique fait passer uniquement un courant électrique entre les jambes de l'utilisateur pour mesurer l'impédance du bas du corps et extrapole la composition corporelle globale, un pèse-personne avec poignée présente l'avantage de pouvoir faire passer un courant entre les mains et les pieds, permettant ainsi de mesurer l'impédance du corps entier, notamment par segment, et d'améliorer la détermination composition corporelle. Cette impédancemétrie est connue sous le nom d'impédancemétrie segmentale. On peut notamment citer comme exemple de pèse-personne impédancemétrique classique Withings BodySmart^{™} et Withings BodyComp^{™} et comme exemple de pèse-personne impédancemétrique à poignée Withings BodyScan^{™} toutes mises dans le commerce en 2023. On peut également citer comme exemple de pèse-personnes impédancemétriques à poignée les pèse-personnes OMRON HBF-510, Huawei Scale 3 Pro, Tanita 780 MC ou encore la InBody 1370.

Afin d'obtenir des mesures d'impédance de bonne qualité, en particulier pour les appareils utilisant des hautes fréquences, il est usuellement recommandé à l'utilisateur pendant sa pesée d'écarter légèrement les bras du corps, d'écarter légèrement les jambes et de placer correctement les pieds sur les électrodes de la base. C'est le cas notamment dans les manuels d'utilisation des pèse-personnes précitées. Cette bonne posture de l'utilisateur permet au courant de circuler normalement à travers l'ensemble de la masse corporelle de l'utilisateur, donnant une mesure précise de l'impédance et donc de la composition corporelle.

### Résumé de l'invention

Toutefois, ces recommandations d'utilisation ne suffisent pas pour garantir une bonne posture de l'utilisateur à chaque pesée et ainsi des mesures fiables dans le temps. En effet, les utilisateurs ne respectent pas tout le temps ces recommandations et il a été remarqué que si les bras de l'utilisateur touchent le tronc (hanches, ventre, etc.) ou les jambes, un court-circuit partiel est créé sur le trajet du courant électrique entre les électrodes. Cela est particulièrement problématique lorsque l'utilisateur est nu ou en sous-vêtements lors de sa mesure. Le courant ne traverse alors pas complètement le corps comme attendu et la détermination de la composition corporelle peut en être affectée. En effet l'algorithme utilisé est conçu pour des chemins électriques prédéterminés, de sorte que tout chemin autre peut provoquer des mesures non exploitables.

La présente description propose donc un dispositif de mesure de composition corporelle permettant une mesure de composition corporelle de l'utilisateur plus fiable et plus reproductible.

Selon un aspect, la présente description concerne une méthode de mesure de composition corporelle comprenant:
- mesure d'une impédance sur un utilisateur à une fréquence (Z2),
- détermination d'une donnée d'impédance (Z2/Z1) à partir de la mesure d'impédance,
- comparaison de cette donnée d'impédance mesurée à une donnée de référence (Se1, Se2),
- en fonction de la comparaison, génération d'un signal d'erreur.

Dans un mode de réalisation, la méthode comprend une association d'une probabilité d'erreur au signal d'erreur, en fonction de la comparaison.

La fréquence est typiquement comprise entre 40 kHz et 5000 kHz, notamment 250 kHz.

Dans un mode de réalisation, la mesure d'impédance est une mesure d'impédance d'un segment de l'utilisateur et la donnée de référence est une donnée de référence d'un segment de référence.

Dans une variante, le segment mesuré est un segment identique au segment de référence.

Dans une variante, le segment mesuré est un segment similaire ou différent du segment de référence, par exemple un segment symétrique du segment mesuré (par exemple segment gauche pour segment droite).

Dans une variante, le segment mesuré est un segment différent du segment de référence, par exemple le segment mesuré est un membre et le segment de référence est un arc-de-jambe.

Dans un mode de réalisation, la donnée de référence est obtenue à partir d'au moins une mesure d'impédance sur le même utilisateur.

Dans une variante, la donnée de référence a été préalablement obtenue, par exemple à partir d'un historique de mesure d'impédance préalablement obtenu sur l'utilisateur.

Dans une variante, la donnée de référence est obtenue concomitamment à la mesure d'impédance, c'est-à-dire lors d'une même session de mesure.

Dans un mode de réalisation, la mesure d'une impédance comprend des mesures d'impédance sur un utilisateur (U) à au moins deux fréquences différentes (F1, F2, F3), dont une première fréquence (F1) (par exemple comprise entre 1 et 10kHz) et une deuxième fréquence (F2) plus élevée que la première fréquence (F1) (par exemple comprise entre 40 kHz et 5000 kHz).

Dans une variante, la donnée d'impédance comprend une combinaison de l'impédance mesurée à la première fréquence et de l'impédance mesurée à la deuxième fréquence. La combination peut comprend une normalisation de la mesure d'impédance à la deuxième fréquence par la mesure d'impédance à la première fréquence. Par exemple, la normalisation est un ratio ou une différence normalisée.

Dans une variante, la donnée d'impédance comprend les mesures d'impédances à la première fréquence et à la deuxième fréquence de l'utilisateur, la donnée de référence comprend des mesures préalablement obtenues sur l'utilisateur à la première fréquence et à la deuxième fréquence et la comparaison comprend la comparaison des données d'impédances aux données de référence, fréquence par fréquence.

La méthode peut en outre comprendre une étape de génération de données impédancemétrie et l'affichage de telles données.

Selon un aspect, l'invention concerne aussi un dispositif de mesure adaptée pour mettre en oeuvre la méthode précédente.

Selon un aspect, dans un mode de réalisation, la présente description concerne un dispositif de mesure de composition corporelle configuré pour : effectuer des mesures d'impédance sur un utilisateur à au moins deux fréquences différentes, dont une première fréquence F1 et une deuxième fréquence F2 plus élevée que la première fréquence; comparer l'impédance mesurée à la première fréquence et l'impédance mesurée à la deuxième fréquence; générer un signal d'erreur sur la base de la comparaison de l'impédance mesurée à la première fréquence et de l'impédance mesurée à la deuxième fréquence.

En effet, les inventeurs ont remarqué qu'en comparant l'impédance mesurée à une première fréquence, notamment une faible fréquence, et l'impédance mesurée à une deuxième fréquence, notamment une plus haute fréquence, il était possible de détecter une posture inappropriée de l'utilisateur, notamment un contact des bras de l'utilisateur avec le tronc et/ou les jambes ou un contact entre les jambes. Les inventeurs ont notamment remarqué que l'impédance à haute fréquence a tendance à décroître assez fortement en cas de posture inappropriée, au contraire de l'impédance à plus basse fréquence qui est moins impactée. En effet, un courant haute fréquence passe plus facilement d'un membre à un autre en cas de contact peau-peau, contrairement à un courant basse fréquence. Ainsi, la comparaison de l'impédance du corps mesurée à la première fréquence et de l'impédance du corps mesurée à la deuxième fréquence permet de détecter un court-circuit partiel du courant à fréquence élevée et donc de détecter un contact peau-peau, qui peut être révélateur d'une posture de l'utilisateur qui n'est pas adaptée pour la mesure et notamment pour un algorithme de traitement associé. Le dispositif de mesure peut alors rejeter la mesure et il peut fournir, soit via un écran soit via une interface tierce comme un téléphone portable, des recommandations de posture peuvent être rappelées pour les prochaines mesures à l'utilisateurs. Les mesures d'impédancemétrie et donc de composition corporelle sont ainsi fiabilisées du fait de la détection des mauvaises postures lors des mesures. L'utilisation de l'impédance mesurée à la fréquence F1 permet en outre de normaliser la mesure d'impédance mesurée à la fréquence F2 par une donnée qui, premièrement vient du même utilisateur et, deuxièmement, est supposée correcte, car il y a peu de court-circuit à la fréquence F1. Par conséquent, l'utilisation des données d'impédance à F1 et F2 permet de s'affranchir d'une calibration sur l'utilisateur : le dispositif de mesure est capable de générer un signal d'erreur, c'est-à-dire de détecter une posture qui n'a pas appropriée pour une mesure de composition corporelle (notamment segmentale), dès la première session de mesure par l'utilisateur.

Dans un mode de réalisation, le dispositif de mesure est configuré pour : en réponse à l'absence de génération de signal d'erreur, déterminer au moins une donnée de composition corporelle en en fonction de l'impédance à la première fréquence et de l'impédance à la deuxième fréquence.

Dans un mode de réalisation, le dispositif de mesure est configuré pour : générer une notification pour l'utilisateur, la notification portant sur une erreur et/ou une posture inappropriée pour l'utilisateur, sur la base du signal d'erreur, notamment un contact de peau entre deux parties du corps de l'utilisateur.

Dans un mode de réalisation, une mesure d'impédance est une mesure d'impédance d'au moins une partie du corps de l'utilisateur

Dans un mode de réalisation, la comparaison des impédances est la comparaison des modules d'impédances.

Dans un mode de réalisation, la comparaison des impédances est la comparaison de la partie résistive des impédances.

Dans un mode de réalisation, le dispositif de mesure comprend : au moins une paire d'électrodes d'injection ; une source de courant alternatif configuré pour injecter un courant dans une portion du corps de l'utilisateur entre la paire d'électrodes d'injection ; au moins une paire d'électrodes de mesure ; un voltmètre configuré pour mesurer une différence de potentiel de la portion du corps de l'utilisateur entre la paire d'électrodes de mesure.

Dans un mode de réalisation, le dispositif de mesure est configuré pour injecter le courant et/ou mesurer une différence de potentiel uniquement par les pieds et/ou les mains et plus précisément par les paumes des mains, les doigts et par le dessous des pieds.

Dans un mode de réalisation, le dispositif de mesure est configuré pour comparer l'impédance mesurée à la première fréquence et l'impédance mesurée à la deuxième fréquence au niveau d'un même segment de l'utilisateur.

Dans un mode de réalisation, le dispositif de mesure comprend en outre un capteur de poids.

Dans un mode de réalisation, le dispositif de mesure comprend une base apte à recevoir les pieds de l'utilisateur

Dans un mode de réalisation, le dispositif de mesure comprend une poignée apte à recevoir les mains de l'utilisateur.

Dans un mode de réalisation, le dispositif de mesure est configuré, en réponse à la génération d'un signal d'erreur, pour : rejeter la mesure d'impédance, et/ou afficher un message à destination de l'utilisateur, et/ou relancer une nouvelle mesure d'impédance.

Dans un mode de réalisation, la comparaison est la comparaison d'un ratio entre l'impédance mesurée à la première fréquence et l'impédance mesurée à la deuxième fréquence avec au moins une valeur seuil d'erreur.

Dans un mode de réalisation, la ou chaque valeur seuil d'erreur est un seuil d'erreur prédéterminé, par exemple obtenu par analyse d'une pluralité de mesures d'impédance préalables sur plusieurs utilisateurs.

Dans un mode de réalisation, la ou chaque valeur seuil d'erreur est propre à l'utilisateur, en particulier fonction d'un ratio d'impédances de référence propre à l'utilisateur, le ratio d'impédances de référence étant par exemple un ratio entre une impédance de référence à la première fréquence et une impédance de référence à la deuxième fréquence mesurées préalablement sur l'utilisateur.

Dans un mode de réalisation, la ou chaque valeur seuil d'erreur est déterminée en fonction d'un historique de ratio d'impédances propre à l'utilisateur, l'historique de ratio d'impédances étant par une moyenne d'au moins deux ratios entre une impédance à la première fréquence et une impédance à la deuxième fréquence mesurées précédemment sur l'utilisateur.

Dans un mode de réalisation, la comparaison comprend une comparaison additionnelle du ratio entre l'impédance à la première fréquence et l'impédance à la deuxième fréquence avec au moins une valeur seuil d'incertitude, et dans lequel le dispositif de mesure est configuré pour générer un signal d'alerte sur la base de la comparaison additionnelle

Dans un mode de réalisation, le dispositif de mesure est configuré pour: effectuer une mesure d'impédance à au moins trois fréquences, dont la première fréquence, la deuxième fréquence et une troisième fréquence comprise entre la première fréquence et la deuxième fréquence; comparer l'impédance mesurée à la première fréquence et l'impédance mesurée à la deuxième fréquence; comparer l'impédance mesurée à la première fréquence et l'impédance mesurée à la troisième fréquence; générer un signal d'erreur sur la base de la comparaison de l'impédance à la première fréquence et de l'impédance à la deuxième fréquence et la comparaison de l'impédance à la première fréquence et de l'impédance à la deuxième fréquence.

Dans un mode de réalisation, la première fréquence est comprise entre 1 kHz et 10 kHz, notamment 5 kHz.

Dans un mode de réalisation, la troisième fréquence est comprise entre 10 kHz et 100 kHz, notamment 50 kHz.

Dans un mode de réalisation, la deuxième fréquence est comprise entre 40 kHz et 5000 kHz, notamment 250 kHz.

Dans un mode de réalisation, le dispositif de mesure est configuré pour effectuer une mesure d'impédance à une pluralité de fréquences, la première fréquence étant la plus petite fréquence disponible parmi la pluralité de fréquences, la deuxième fréquence étant la plus grande fréquence disponible parmi la pluralité des fréquences.

Dans un mode de réalisation, les mesures d'impédance effectuées à au moins deux fréquences sont effectuées entre les mêmes électrodes.

Dans un mode de réalisation, le dispositif de mesure est configuré pour comparer les impédances mesurées entre les mêmes électrodes.

La présente description concerne également une méthode de mesure comprenant au moins les étapes successives suivantes : mesures d'impédance sur un utilisateur à au moins deux fréquences différentes , dont une première fréquence et une deuxième fréquence plus élevée que la première fréquence; comparaison de l'impédance mesurée à la première fréquence et l'impédance mesurée à la deuxième fréquence; génération d'un signal d'erreur sur la base de la comparaison de l'impédance mesurée à la première fréquence et de l'impédance mesurée à la deuxième fréquence.

Dans un mode de réalisation, la méthode de mesure comprend en outre, en l'absence de signal d'erreur, la détermination d'au moins une donnée de composition corporelle en en fonction de l'impédance à la première fréquence et de l'impédance à la deuxième fréquence.

La présente description concerne en outre un produit programme d'ordinateur comprenant des instructions qui, lorsque le programme est exécuté par un ordinateur, conduisent celui-ci à mettre en oeuvre la méthode de mesure tel que définie ci-dessus.

Dans un mode de réalisation, la présente description concerne un dispositif de mesure impédancemétrique configuré pour : effectuer des mesures d'impédance sur un utilisateur à au moins deux fréquences différentes, dont une première fréquence et une deuxième fréquence plus élevée que la première fréquence; comparer l'impédance mesurée à la première fréquence et l'impédance mesurée à la deuxième fréquence; générer un signal d'erreur sur la base de la comparaison de l'impédance mesurée à la première fréquence et de l'impédance mesurée à la deuxième fréquence.

### Présentation des figures

D'autres caractéristiques, détails et avantages apparaîtront à la lecture de la description détaillée ci-après, et à l'analyse des dessins annexés, sur lesquels :
- [FIG. 1] : la Figure 1 présente deux vues schématiques d'un dispositif de mesure à quatre électrodes,
- [FIG. 2] : la Figure 2 présente une vue schématique d'un dispositif de mesure à huit électrodes,
- [FIG. 3] : la Figure 3 présente une vue en perspective d'un mode de réalisation d'un dispositif de mesure à quatre électrodes sous forme d'un pèse-personne,
- [FIG. 4] : la Figure 4 présente une vue en perspective d'un mode de réalisation d'un dispositif de mesure à huit électrodes, (a) avec une poignée en position retractée, et (b) avec une poignée en position déployée, sous forme d'un pèse-personne
- [FIG. 5] : la Figure 5 présente une vue schématique de la station de mesure et de son environnement ;
- [FIG. 6] : la Figure 6 présente une vue de face (a) et une vue de côté (b) d'une recommandation de posture pendant une mesure avec un dispositif de mesure des Figures 3 et 4,
- [FIG. 7] : la Figure 7 illustre le fonctionnement de l'analyse d'impédancemétrie segmentale,
- [FIG. 8] : la Figure 8 présente deux vues schématiques d'une mesure avec un dispositif de mesure, sans (a) et avec (b) un court-circuit partiel,
- [FIG. 9] : la Figure 9 présente une distribution de ratio d'impédances d'une pluralité d'utilisateurs, mesurées avec un dispositif de mesure à des fréquences de 250 kHz et 5 kHz, respectivement dans le bras gauche (a), dans le tronc (b) et dans la jambe gauche (c),
- [FIG. 10] : la Figure 10 présente une distribution de ratios d'impédances d'une pluralité d'utilisateurs, mesurées avec un dispositif de mesure à des fréquences de 50 kHz et 5 kHz, respectivement dans le bras gauche (a), dans le tronc (b) et dans la jambe gauche (c),
- [FIG. 11] : la Figure 11 présente une distribution de ratio d'impédances avec deux valeurs seuil d'erreur et deux valeurs seuil d'incertitude,
- [FIG. 12] : la Figure 12 présente une distribution de ratio d'impédances dans le corps complet pour les hommes et pour les femmes,
- [FIG. 13] : la Figure 13 présent un organigramme d'une méthode de mesure impédancemétrique,
- [FIG. 14] : la Figure 14 présente des pourcentages de masse grasse d'une pluralité d'utilisateurs en fonction du pourcentage de masse grasse obtenu dans un cas de référence pour le bras gauche (lam), le torse (torso), le bras droit (ram), la jambe gauche (Ilg), le corps complet (wbd) et la jambe droite (rlg), sans détection de posture inappropriée,
- [FIG. 15] : la Figure 15 présente des pourcentages de masse grasse d'une pluralité d'utilisateurs en fonction du pourcentage de masse grasse obtenu dans un cas de référence pour le bras gauche (lam), le torse (torso), le bras droit (ram), la jambe gauche (Ilg), le corps complet (wbd) et la jambe droite (rlg), avec détection et rejet des mauvais postures,
- [FIG. 16] : la Figure 16 présente deux graphiques représentant deux ratios d'impédances l'un en fonction de l'autre d'une pluralité d'utilisateurs sans (a) et avec (b) détection d'erreur.

### Description détaillée

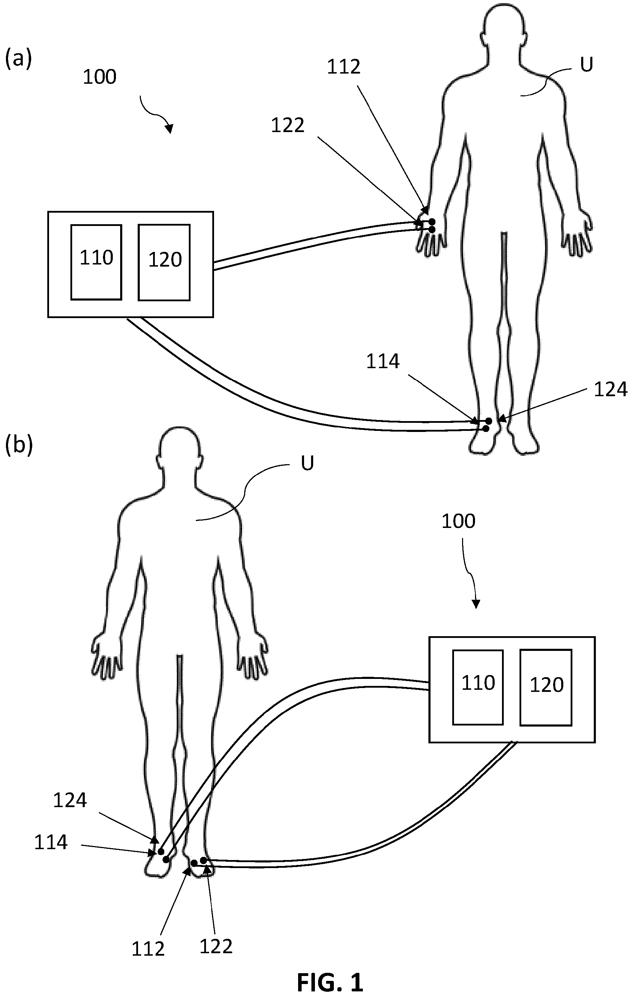

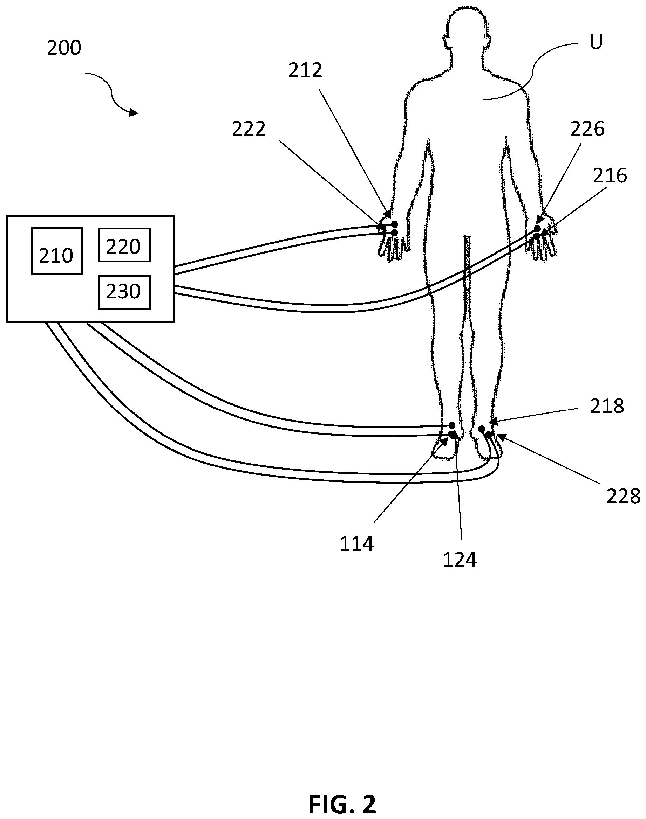

Deux variantes de dispositifs de mesure 100, 200 sont représentés schématiquement sur les Figures 1 et 2. Le dispositif de mesure 100, 200 comprend une source de courant alternatif 110, 210 et au moins deux électrodes d'injection 112, 114, 212, 214 reliées à la source de courant alternatif 110, 210 et configurées pour injecter un courant alternatif dans le corps d'un utilisateur U. Dans tout le présent document, le terme de « courant » signifie « courant électrique ». Le dispositif de mesure 100, 200 comprend en outre un voltmètre 120, 220 et au moins deux électrodes de mesure 122, 124, 222, 224 reliées au voltmètre 120, 220 et configurées pour récupérer un potentiel du corps humain.

La source de courant alternatif 110, 210 est configurée pour injecter un courant dans une portion du corps de l'utilisateur entre chaque paire des au moins deux électrodes d'injection 112, 114, 222, 224 et le voltmètre 120, 220 est configuré pour mesurer une différence de potentiel de la portion de l'utilisateur entre chaque paire des au moins deux électrodes de mesure 122, 124, 222, 224.

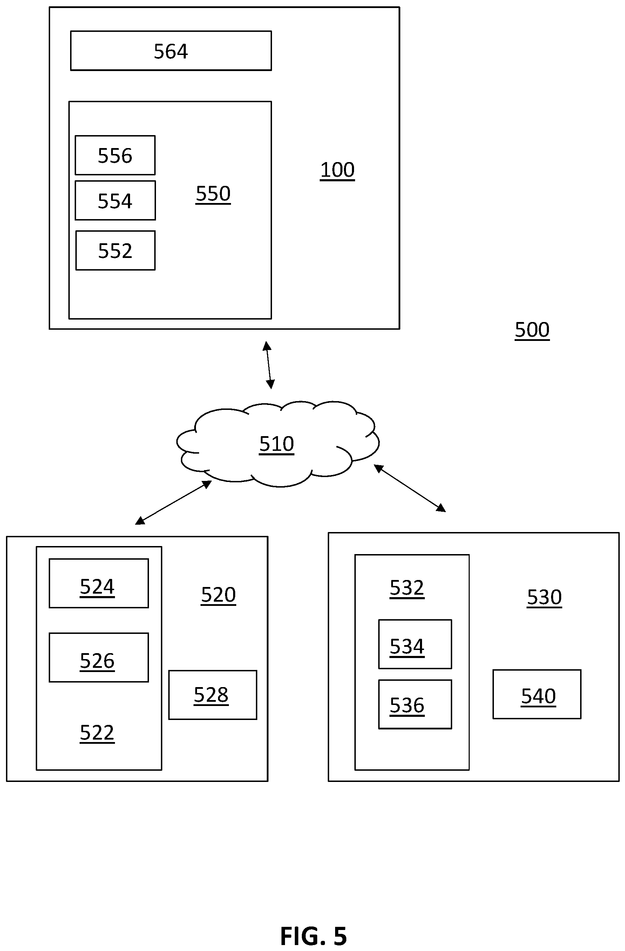

Le dispositif de mesure 100, 200 comprend en outre une circuiterie de contrôle 550, représentée sur la Figure 5, configurée pour contrôler la source de courant 110, 210 et le voltmètre 120, 220 et d'obtenir ainsi des valeurs d'impédances d'une ou plusieurs parties du corps de l'utilisateur. La circuiterie de contrôle 550 est notamment configurée pour déterminer au moins une donnée relative à la composition corporelle de l'utilisateur U grâce à un algorithme de composition corporelle, à partir des mesures d'impédance et par exemple du profil de l'utilisateur comprenant notamment son sexe et son âge. Par composition corporelle, il est signifié de façon non limitative, en absolu ou en pourcentage : une masse grasse, une masse hydrique, une masse musculaire, une masse osseuse.

L'algorithme de composition corporelle est typiquement un algorithme embarqué dans le dispositif de mesure 100, 200.

En particulier, la source de courant alternatif 110, 210 est configurée pour injecter le courant électrique alternatif à faible intensité, notamment inférieur à 2,5 mA.

La source de courant alternatif 110, 210 est notamment configurée pour injecter le courant électrique à une fréquence comprise entre 1 kHz et 5000 kHz. Comme cela sera expliqué plus en détail par la suite, la source de courant alternatif 110, 210 est configurée pour injecter un courant électrique à au moins deux fréquences différentes, c'est-à-dire deux courants électriques qui ont chacun une fréquence différente.

Par analyse d'impédance à une fréquence donnée, il est signifié à un courant alternatif à la fréquence donnée.

Le dispositif de mesure 100, 200 est ainsi configuré pour mesurer les valeurs d'impédance du corps, ou d'une portion du corps, à au moins deux fréquences de courant différentes, par exemple trois fréquences de courant. Dans un mode de réalisation, le dispositif de mesure 100, 200 est configuré pour mesurer les valeurs d'impédance pour moins de 25 fréquences de courant, notamment moins de 10 fréquences de courant, notamment moins de 5 fréquences de courant, et notamment 3 fréquences de courant.

Les contraintes d'un usage à domicile, en autonomie par un utilisateur comportent des contraintes fortes sur le dispositif de mesures. En effet, ce dernier fonctionne sur batterie et son coût de fabrication doit être compatible avec la production de masse (nature des composants, disponibilité et prix).

Enfin, le but de l'impédancemétrie étant ici d'obtenir des informations de compositions corporelles, la multiplication des fréquences n'apporte pas d'avantage significatif au-delà d'un nombre de fréquences. L'analyse impédancemétrique à trois fréquences représente déjà une approche avancée pour la composition corporelle. L'ajout de fréquence supplémentaire génère notamment une complexité algorithmique supplémentaire.

L'approche multifréquences offre plusieurs avantages. En particulier, le dispositif de mesure 100, 200 est plus précis. En effet, chaque courant traverse les tissus corporels de l'utilisateur U de manière différente en fonction de sa fréquence. Les courant à fréquences basses ont tendance à se pénétrer uniquement les fluides extracellulaires, tandis que les courants à fréquences plus élevées peuvent traverser les membranes cellulaires et mesurer les fluides intracellulaires. Une approche multifréquence permet donc une mesure plus précise de la composition corporelle globale.

Le dispositif de mesure 100, 200 est également configuré pour différencier la quantité de fluides intracellulaires et extracellulaires. En effet, aux basses fréquences (par exemple autour de 50 kHz), le courant ne pénètre pas ou peu les cellules : il ne traverse que les fluides extracellulaires (plasma sanguin, liquide interstitiel). A l'inverse, aux hautes fréquences (par exemple autour de 250 kHz), le courant traverse à la fois les fluides extra et intracellulaires. En analysant les différences d'impédance entre les basses et hautes fréquences, il est ainsi possible de déterminer la quantité respective de fluides extra et intracellulaires dans le corps.

Dans un mode de réalisation, dit à quatre électrodes, illustré sur la Figure 1, les deux électrodes d'injection 112, 114 reliées à la source de courant alternatif 110 sont configurée pour être en contact avec respectivement deux membres différents de l'utilisateur U. De la même façon, les deux électrodes de mesure 122, 124 reliées au voltmètre 120 sont configurés pour être en contact avec respectivement deux membres différents. Les différents agencements possibles sont connus et permettent de mesurer l'impédance Z des différents segments de l'utilisateur.

Par la suite, on entend par segment une partie du corps humain pour laquelle l'impédance est calculée, notamment le bras gauche, le bras droit, la jambe gauche, la jambe droite, l'arc de jambes, le tronc.

Par exemple, la Figure 1 (a) illustre un agencement avec l'une des électrodes de mesure 122 en contact avec une main de l'utilisateur U et l'autre électrode de mesure 124 en contact avec un pied de l'utilisateur U. Dans ce mode de réalisation, le courant électrique traverse le corps depuis une main, par exemple la main droite dans cet exemple, vers un pied, par exemple le pied droit dans cet exemple, et l'impédance mesurée est donc celle du bras (ici droit), du tronc et de la jambe (ici droite) combinées. Ce mode de réalisation offre ainsi une estimation globale de la composition corporelle de l'utilisateur.

Par exemple, la Figure 1 (b) illustre un agencement avec l'une des électrodes de mesure 122 en contact avec un pied de l'utilisateur U et l'autre électrode de mesure 124 en contact avec l'autre pied de l'utilisateur U, et pareillement pour les électrodes de mesure 112, 114. Dans cet agencement, le courant électrique traverse le corps depuis un pied à l'autre pied et l'impédance mesurée est donc celle des deux jambes combinées. Cet agencement offre ainsi une estimation globale de la composition corporelle du bas du corps de l'utilisateur. Avec le dispositif de mesure 100, il est nécessaire de déplacer les électrodes sur les autres membres pour faire une analyse de composition corporelle segmentale complète.

Dans un mode de réalisation, dit à huit électrodes, illustré sur la Figure 2, le dispositif de mesure 200 comprend quatre électrodes d'injection 212, 214, 216, 218 agencées par paires et reliées à la source de courant alternatif 210, notamment par un commutateur 230. Les électrodes d'injection 212, 216, 214, 218 sont configurées pour être en contact avec respectivement les deux mains et les deux pieds de l'utilisateur U. Plus spécifiquement, le commutateur permet de relier deux électrodes (formant la paire) parmi quatre électrodes d'injection 212, 214, 216, 218 à la source de courant alternatif 210.

Le dispositif de mesure 200 comprend en outre quatre électrodes de mesure 222, 224, 226, 228 agencées par paires et reliées au voltmètre 220, notamment par le commutateur 230. Deux électrodes de mesure 222, 226 sont configurées pour être en contact avec respectivement chacune des mains de l'utilisateur et les deux autres électrodes de mesure 224, 228 sont configurées pour être en contact avec respectivement chacun des deux pieds de l'utilisateur U. Plus spécifiquement, le commutateur 230 permet de relier deux électrodes (formant la paire) parmi quatre électrodes de mesure 222, 224, 226, 228 au voltmètre 220.

Le dispositif de mesure 200 à huit électrodes permet d'obtenir une composition corporelle dite segmentale de l'utilisateur en une seule manipulation du dispositif de mesure 200. En effet, un dispositif de mesure à huit électrodes permet de mesurer l'impédance à travers différents segments du corps notamment les bras, les jambes et le tronc séparément. A l'aide du commutateur qui permet de réaliser toutes les paires possibles d'électrodes d'injection et d'électrode de mesure, l'impédance de chacun des segments du corps peut être calculée. Par exemple, l'impédance des bras peut être déterminée en faisant passer le courant entre l'électrode d'une main à l'électrode de l'autre main. Par exemple, l'impédance d'un seul bras peut aussi par exemple être mesurée en faisant passer le courant entre un pied et une main et en mesurant la tension entre les 2 mains. Par exemple, l'impédance d'un seul bras peut aussi par exemple être mesurée en faisant passer le courant entre les deux mains et en mesurant la tension entre le pied et la main. Par exemple, l'impédance des jambes peut être déterminée en faisant passer le courant entre l'électrode d'un pied à l'électrode de l'autre pied. Par exemple, l'impédance d'une seule jambe peut aussi par exemple être mesurée en faisant passer le courant entre un pied et une main et en mesurant la tension entre les deux pieds. L'impédance du tronc peut être déduite des impédances du corps entier dont on soustrait l'impédances des bras et des jambes.

En particulier, le dispositif de mesure 200 comprend des électrodes fixes, en ce sens où elles ne viennent pas successivement en contact de parties différentes de l'utilisateur U en fonction des manipulations.

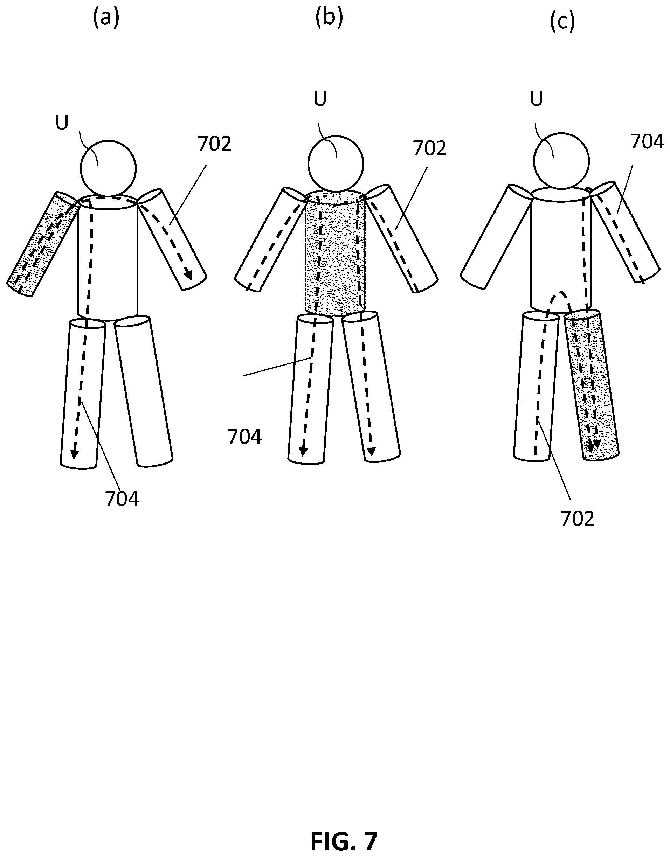

La figure 7 illustre le fonctionnement de l'analyse d'impédancemétrie segmentale, et donc d'analyse de composition segmentale : les lignes 702 représentent des lignes de courants, entre deux électrodes d'injection (non représentées ici) et les lignes 704 représentent les lignes de tension, entre deux électrodes de mesure (non représentées). L'impédance mesurée correspond à l'impédance du segment (bras, jambes, tronc, etc.) par lequel passe une ligne de courant et une ligne de tension (le segment est la portion grisée sur la figure). A titre d'exemple non limitatif, la configuration de la figure 7(a) permet de mesurer l'impédance du bras gauche, celle de la figure 7(b) du tronc et celle de la figure 7(c) de la jambe droite.

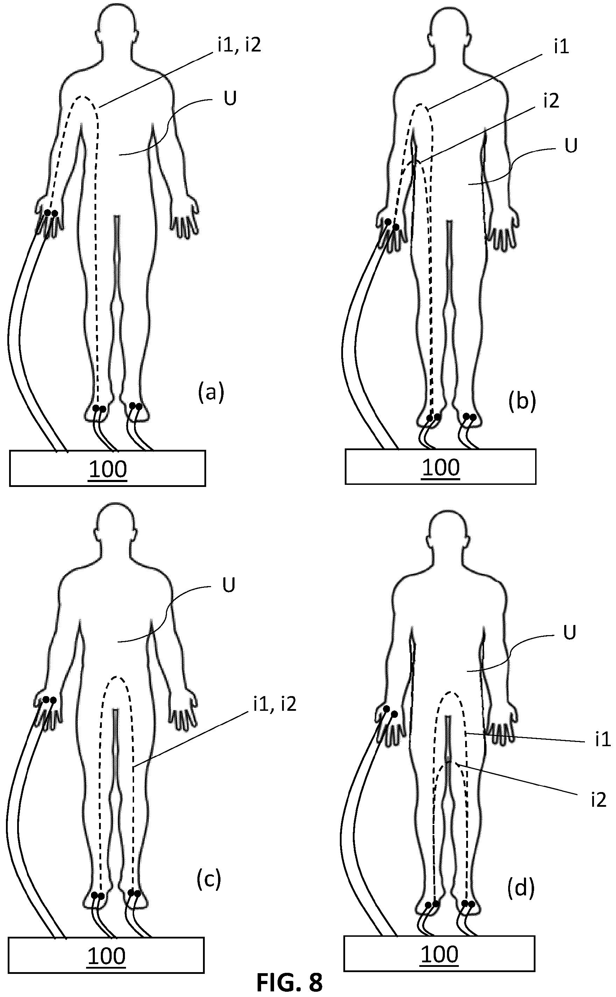

Pour effectuer une mesure de composition, le commutateur relie les électrodes adéquates à la source de courant et de tension pour faire les mesures illustrées en figure 8.

Typiquement, six mesures d'impédancemétrie sont effectuées pour obtenir l'impédance de : bras gauche, bras droit, jambe gauche, jambe droite, tronc, et arc de jambe. L'ensemble de ces six mesures prend moins de 5s, voire moins de 3s. Plus généralement, une analyse d'impédancemétrie pour composition corporelle segmentale prend moins de 5s.

### Pèse-personnes impédancemétrique

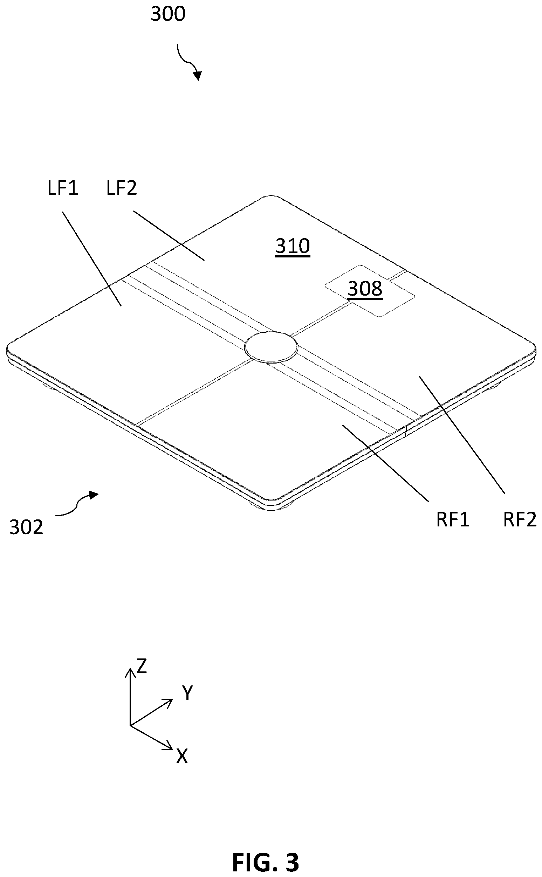

La Figure 3 illustre un dispositif de mesure 300, qui est un mode de réalisation particulier du dispositif de mesure 100, notamment en ce qu'il est sous la forme d'un pèse-personne impédancemétrique dit "classique".

Le dispositif de mesure 300 se présente essentiellement sous la forme d'une base 302 sur laquelle un utilisateur peut poser ses pieds, par exemple à plat. L'utilisateur peut être debout sur la base 302 ou assis sur une chaise.

Le dispositif de mesure 300 peut comprendre en outre des capteurs de poids, comme des cellules de charge, apte à mesurer un poids de l'utilisateur. Les capteurs de poids peuvent permettent aussi d'effectuer un BCG (ballistocardiogramme), c'est-à-dire une mesure de variation du poids sous l'effet de l'éjection du sang du cœur. Le dispositif de mesure 300 se présente alors sous la forme d'un pèse-personne impédancemétrique.

La base 302 peut comprendre un affichage 308, notamment un écran ou un affichage à LED ou à encre électronique, pour afficher des informations à destination de l'utilisateur.

La base 302 comprend en outre une plaque de mesure 310 apte à recevoir les pieds de l'utilisateur. La plaque de mesure 310 transmet le poids de l'utilisateur vers les capteurs de poids.

Typiquement, afin d'être capable d'effectuer une mesure d'impédancemétrie complète en minimisant les actions de la part de l'utilisateur U, le dispositif de mesure 300 comprend plusieurs électrodes, et notamment un couple d'électrode par pied de l'utilisateur. Concrètement, cela signifie que la base 302 comprend deux électrodes gauches LF1, LF2 (destinées à être au contact du pied gauche de l'utilisateur, plus précisément le dessous du pied), deux électrodes droits RF1, RF2 (destinées à être au contact du pied droite de l'utilisateur, plus précisément le dessous du pied). Les électrodes LF1, LF2, RF1, RF2 correspondent aux électrodes 112, 114, 116, 118. Les électrodes de la base 302 sont typiquement montées sur la plaque de mesure 310. Comme les électrodes LF1, LF2, RF1, RF2 peuvent avoir différentes fonctions pour différentes mesures, le dispositif de mesure 300 comprend un commutateur (non visible) qui permet de connecter ou déconnecter les électrodes à différents composants (source de courant, voltmètre, source de tension, etc.).

Grâce à cet arrangement à quatre électrodes, il est possible d'effectuer une analyse de composition corporelle "arc de jambes" de l'utilisateur de façon rapide (par exemple moins de 5s, voire 2s) et simple : l'utilisateur U n'a qu'à monter sur la base 302 avec ses deux pieds.

En utilisation normale, les électrodes du dispositif 300 sont conçues pour être au contact du dessous du pied uniquement.

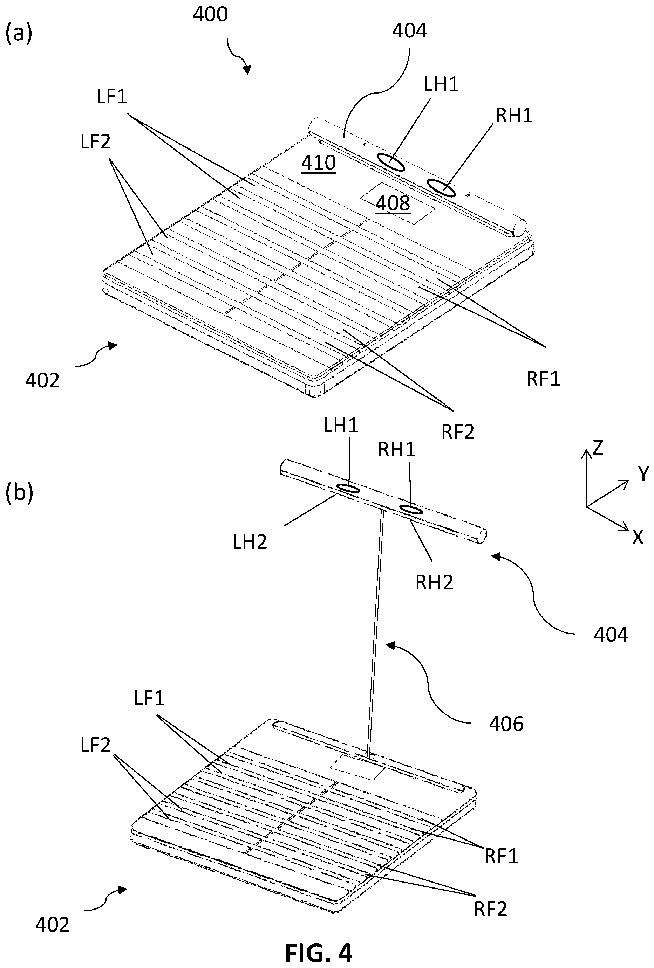

La Figure 4 illustre un dispositif de mesure 400, qui est un mode de réalisation particulier du dispositif de mesure 200, notamment en ce qu'il est sous la forme d'un pèse-personne impédancemétrique avec poignée.

Le dispositif de mesure 400 se présente essentiellement sous la forme d'une base 402 sur laquelle un utilisateur peut poser ses pieds, par exemple à plat. L'utilisateur peut être debout sur la base 402 ou assis sur une chaise.

Le dispositif de mesure 400 peut comprendre en outre des capteurs de poids, comme des cellules de charge, apte à mesurer un poids de l'utilisateur. Les capteurs de poids peuvent permettent aussi d'effectuer un BCG (ballistocardiogramme), c'est-à-dire une mesure de variation du poids sous l'effet de l'éjection du sang du cœur. Le dispositif de mesure 400 se présente alors sous la forme d'un pèse-personne impédancemétrique.

Le dispositif de mesure 400 peut comprendre en outre une poignée 404, apte à être saisie par une moins une main de l'utilisateur. La poignée 404 peut être reliée à la station de mesure par un câble 406 visible sur la Figure 4 (b). Le câble 406 peut se déployer, comme visible sur la Figure 4 (b) et se rétracter, comme visible sur la Figure 4 (a), par exemple s'enrouler et se dérouler à l'intérieur de la base 402.

La base 402 peut comprendre un affichage 408, notamment un écran ou un affichage à LED ou à encre électronique, pour afficher des informations à destination de l'utilisateur.

La base 402 comprend en outre une plaque de mesure 410 apte à recevoir les pieds de l'utilisateur. La plaque de mesure 410 transmet le poids de l'utilisateur vers les capteurs de poids.

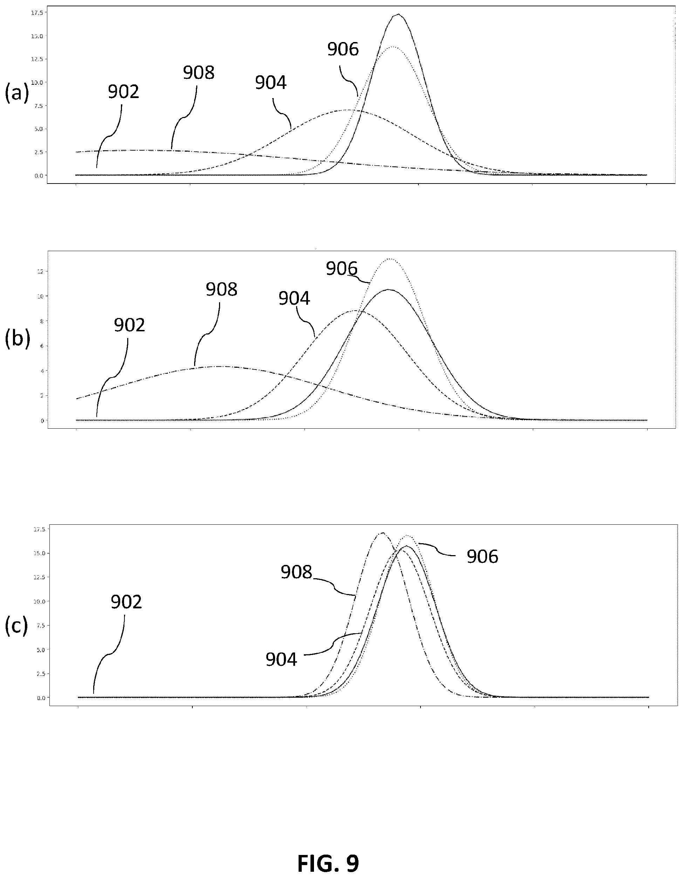

Typiquement, afin d'être capable d'effectuer une mesure d'impédancemétrie complète en minimisant les actions de la part de l'utilisateur U, le dispositif de mesure 400 comprend plusieurs électrodes, et notamment un couple d'électrode par membre de l'utilisateur. Concrètement, cela signifie que la base 402 comprend deux électrodes gauches LF1, LF2 (destinées à être au contact du pied gauche de l'utilisateur, plus précisément le dessous du pied), deux électrodes droits RF1, RF2 (destinées à être au contact du pied droite de l'utilisateur , plus précisément le dessous du pied) et la poignée comprend deux électrodes gauches LH1, LH2 (destinées à être au contact de la main gauche de l'utilisateur, plus précisément la paume ou les doigts) et deux électrode droits RH1, RH2 (destinées à être au contact de la main droite de l'utilisateur, plus précisément la paume ou les doigts). Les électrodes LF1, LF2, RF1, RF2, LH1, LH2, RH1, RH2 correspondent aux électrodes 212, 214, 216, 218, 222, 224, 226, 228. Le document WO2023126220 décrit avec plus de détail l'architecture électronique et les variantes possibles (voir en particulier les figures 9 à 18), notamment pour effectuer les six configurations mentionnées en relation avec la figure 7.

Grâce à cet arrangement à huit électrodes, toutes les configurations possibles pour une analyse de composition segmentales sont possibles de façon rapide (par exemple moins de 5s, voire 2s) et simple : l'utilisateur U n'a qu'à monter sur la base 402 avec ses deux pieds et n'à saisir la poignée 404 avec ses deux mains, puis le commutateur attribue les fonctions d'électrodes d'injection ou de mesure aux électrodes LF1, LF2, RF1, RF2, LH1, LH2, RH1, RH2 pour effectuer la mesure programmée.

Les électrodes de la base 402 sont typiquement montées sur la plaque de mesure 410.

Par exemple, selon des configurations, deux électrodes d'injection 112, 114 sont arrangées sur la plaque de mesure 310 et deux électrodes d'injection 116, 118 sont arrangées sur la poignée 404, ou bien, deux électrodes de mesure 122, 124 sont arrangées sur la plaque de mesure 310 et deux électrodes de mesure 126, 128 sont arrangées sur la poignée 404.

En utilisation normale, les électrodes du dispositif 400 sont conçues pour être au contact du dessous du pied, de la paume de la main, voire des doigts.

Un tel dispositif de mesure 400 est décrit plus en détail dans les documents WO2023126220, US2023210429 et US2023210430.

### Connectivité

La figure 5 illustre une vue schématique de l'architecture globale 500 dans laquelle le dispositif de mesure 100, 200, 300, 400 peut être inséré. Cette architecture globale forme un système comprenant le dispositif de mesure 100, 200, 300, 400. En particulier, le dispositif de mesure 100, 200, 300, 400 peut communiquer avec des dispositifs tiers via un réseau de communication 510, qui est par exemple un réseau sans-fil (notamment un réseau compatible avec au moins l'un des protocoles de communication suivants : Bluetooth, Wi-Fi, cellulaire, etc.). Les dispositifs tiers peuvent comprendre un serveur 520 et un terminal mobile 530 (smartphone, etc.). Le serveur 520 peut comprendre une circuiterie de contrôle 522, incluant un processeur 524 et une mémoire 526, et une interface entrée/sortie (« input/output », I/O) 528, qui permet à la circuiterie de contrôle de recevoir et d'envoyer des données vers le réseau de communication 510. Le terminal mobile 530 peut comprendre une circuiterie de contrôle 532, incluant un processeur 534 et une mémoire 536, un comprendre une interface entrée/sortie (« input/output », I/O) 538, qui permet à la circuiterie de contrôle de recevoir et d'envoyer des données. La mémoire 526 peut stocker l'algorithme de composition corporelle, ainsi que des profils utilisateur correspondant aux utilisateurs associés au dispositif 500. Le serveur 520 est un serveur distant, par exemple situé dans un centre de données (data center). Le terminal mobile 530 comprend en outre une interface d'utilisateur 540 (« user interface », UI) configurée pour afficher des informations à l'utilisateur et lui permettre, le cas échant, d'entrer des informations (comme la taille, le sexe, etc.). En particulier, la circuiterie de contrôle 532 est configurée pour faire fonctionner une application gérant l'environnement du dispositif de mesure 100, 200, 300, 400. Le terminal mobile 530 est un objet personnel de l'utilisateur, généralement à proximité de ce dernier.

La station de mesure 100, 200, 300, 400 peut communiquer avec le serveur 520 et/ou le terminal mobile 530. Dans un mode de réalisation, la station de mesure 100, 200, 300, 400 peut communiquer directement avec le terminal mobile 530, par exemple via Bluetooth ou Bluetooth Low Emission (BLE). Cette communication peut être mise à en oeuvre à l'installation de l'appareil de mesure 100, 200, 300, 400 notamment pour l'appairer avec le terminal mobile 530 et/ou pour configure une connexion au serveur 520 qui ne transite pas par le terminal mobile 530 et/ou en en secours d'une communication avec le serveur 520 défaillante. Dans un mode de réalisation, la station de mesure 100, 200, 300, 400 peut communiquer directement avec le serveur 520, sans transiter par le terminal mobile 530. Cette communication permet à l'utilisateur d'utiliser la station de mesure même sans avoir son terminal mobile 530 à proximité.

La station de mesure 100, 200, 300, 400 comprend elle aussi une circuiterie de contrôle 550 avec un processeur 552 et une mémoire 554, et une interface entrée/sortie 556 (« input/output », I/O), qui permet notamment à la circuiterie de contrôle de recevoir et d'envoyer des données au réseau de communication 510. Le processeur 552 est configuré pour notamment traiter des données obtenues par les électrodes d'injection et de mesure. En particulier, le processeur 552 peut exécuter des instructions d'un programme stocké dans la mémoire 554. La circuiterie de contrôle 550 peut comprendre un microcontrôleur, qui intègre le processeur 552, la mémoire 554 et l'interface entrée/sortie 556. La circuiterie de contrôle 550 peut en outre comprendre un dispositif frontal analogique (« Analog Front End », AFE). Le voltmètre 120, 220 peut être intégré à l'AFE. La circuiterie de contrôle 550 peut aussi comprendre un convertisseur analogique/digital (« Analog to Digital Converters », ADC). La source de courant 110, 210 peut être intégré à l'AFE. Les électrodes d'injection et de mesure sont reliées à la circuiterie de contrôle 550. La station de mesure 100, 200, 300, 400 comprend une batterie 564, apte à alimenter en énergie les différents composants de la station de mesure 100, 200, 300, 400.

### Recommandation de posture

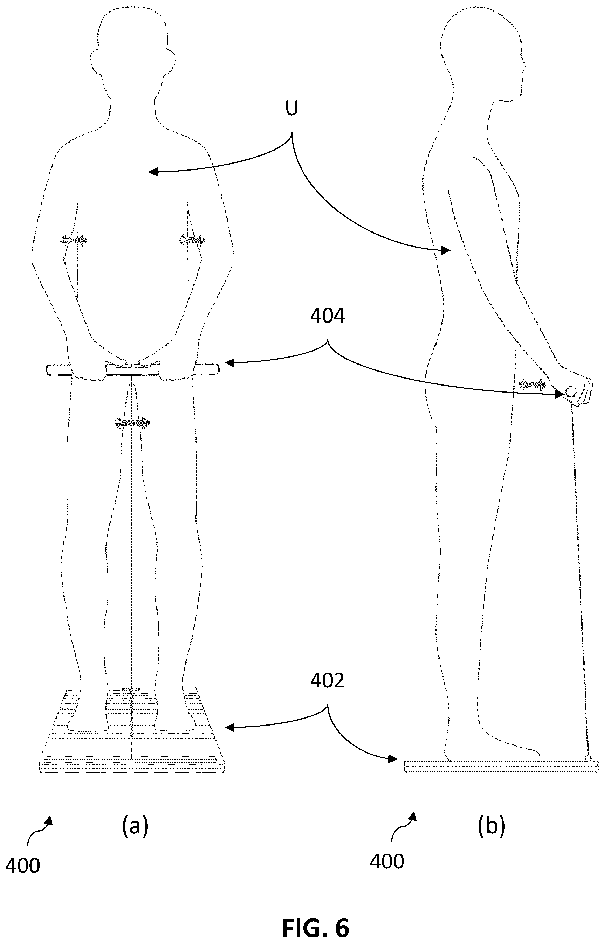

La Figure 6 présente un exemple de posture de l'utilisateur adapté pour obtenir une mesure d'impédance considérée comme fiable. Fiable signifie ici que la posture est adaptée à l'algorithme de calcul d'impédancemétrie.

En particulier, la posture adaptée pour l'utilisateur est une position debout, immobile et détendue. Les pieds doivent être nus et à plat sur les électrodes de la base 402. Dans le cas d'un pèse-personne sans poignée, les jambes sont préférentiellement légèrement écartées. Dans le cas d'un pèse-personne à poignée comme illustré ici, les jambes sont préférentiellement légèrement écartées entre elles et les bras sont éloignés du tronc et des jambes. Les mains saisissent la poignée 404 et sont en contact avec les électrodes de la poignée.

### Détection d'erreur

Le dispositif de mesure 100, 200, 300, 400, 500 est configuré pour effectuer une ou plusieurs mesures d'impédance sur l'utilisateur, par exemple à au moins deux fréquences différentes de courant alternatif. L'impédance est calculée à l'aide de la valeur du courant i injecté par les électrodes d'injection et de la valeur de tension mesurée par les électrodes de mesure.

Par "mesure d'impédance", il est entendu une mesure d'impédance d'une au moins une partie du corps de l'utilisateur, généralement appelé segment (d'où l'appellation de composition segmentale). Dans un mode de réalisation, la mesure d'impédance est la mesure d'impédance d'au moins un segment, par exemple un bras, une jambe ou le tronc. En variante ou en option encore, la mesure d'impédance est la mesure d'impédance de deux segments consécutifs, par exemple un bras et un tronc ou un tronc et une jambe ou deux jambes. En variante ou en option encore, la mesure d'impédance est la mesure d'impédance de chacun des segments (bras, tronc, jambes) de l'utilisateur. En variante ou en option encore, la mesure d'impédance est la mesure d'impédance d'un demi-corps, voire d'un corps complet, c'est-à-dire des mains jusqu'aux pieds.

En particulier, les deux fréquences pour le courant alternatif comprennent notamment une première fréquence F1 et une deuxième fréquence F2. La deuxième fréquence F2 est plus élevée que la première fréquence F1.

En particulier, la deuxième fréquence F2 est au moins cinq fois, par exemple dix fois, ou encore cinquante fois plus élevée que la première fréquence F1.

Dans un mode de réalisation, la première fréquence F1 est une fréquence dit basse fréquence, autrement dit inférieure à 10 kHz et la deuxième fréquence F2 est une fréquence dite haute fréquence, autrement dit supérieure à 40 kHz.

Dans un autre mode de réalisation, le dispositif de mesure 100, 200, 300, 400 est configuré pour effectuer une mesure d'impédance à au moins trois fréquences, dont la première fréquence F1, la deuxième fréquence F2 et une troisième fréquence F3 comprise entre la première fréquence F1 et la deuxième fréquence F2 (F1<F2<F3).

Dans un mode de réalisation, le dispositif de mesure 100, 200, 300, 400 est configuré pour effectuer des mesures d'impédance à une pluralité de fréquences. Par exemple, la première fréquence F1 est la plus petite fréquence disponible par le générateur de courant alternatif parmi la pluralité de fréquences et la deuxième fréquence F2 est la plus grande fréquence disponible par le générateur de courant alternatif parmi la pluralité de fréquences.

La première fréquence F1 est par exemple comprise entre 1 kHz et 10 kHz ; elle peut être fixée à 5 kHz. La troisième fréquence F3 est par exemple comprise entre 10 kHz et 100 kHz ; elle peut être fixée à 50 kH. La deuxième fréquence F2 est par exemple comprise entre 40 kHz et 5000 kHz ; elle peut être fixée par exemple à 250 kHz. Dans le cadre des définitions de la présente description, on a toujours F1<F2<F3.

A partir de l'impédance mesurée, le dispositif de mesure 100, 200, 300, 400, 500 peut déterminer une donnée d'impédance mesurée. La donnée d'impédance mesurée est alors comparée à une donnée de référence pour notamment déterminer si l'impédance mesurée est une donnée fiable ou non.

### Comparaison d'impédances

Un contact peau-peau représente électriquement un ajout d'une capacité (condensateur), en parallèle avec une résistance, un courant à haute fréquence passe plus facilement qu'un courant basse fréquence d'un membre à un autre en cas de contact peau-peau et crée un court-circuit partiel. Par conséquence, comme une partie du courant électrique prend le chemin électrique plus court, l'impédance calculée à haute fréquence (e.g. 250kHz) pour ce membre court-circuité a tendance à être artificiellement plus faible, au contraire de l'impédance à plus basse fréquence qui est moins impactée puisque le courant basse fréquence n'est pas court-circuité (la capacité et la résistance de la peau s'oppose au passage du courant basse fréquence).

La Figure 8 illustre quatre situations (a), (b), (c), (d) pour la circulation de courant avec notamment des courts-circuits. Ces situations ne sont pas limitatives, en ce que notamment le court-circuit peut être généré par autre chose qu'un contact peau-peau et en ce que le court-circuit peut avoir lieu à un autre endroit du corps de l'utilisateur.

En Figure 8(a), le dispositif de mesure injecte successivement deux courants i1 (à la fréquence F1), i2 (à la fréquence F2) entre la main droite et le pied droit pour mesurer l'impédance du côté droit de l'utilisateur U (soit le bras, soit la jambe, soit l'hémicorps droit). L'utilisateur U a une posture considérée comme approprié, avec les bras écartés, ce qui fait que la circulation des courant i1, i2 se fait selon le chemin désiré, c'est-à-dire le long du ou des segments pour lesquels le dispositif de mesure calcule l'impédance. Le dispositif de mesure calcule une impédance Z1a avec le courant i1 et une impédance Z2a avec le courant i2. En pratique, i2 n'est pas nécessairement entièrement bypassé mais une partie seulement du courant i2 peut être court-circuitée.

En Figure 8(b), la configuration est identique à la figure 8(a), sauf que l'utilisateur U a une posture qui n'est pas considéré comme appropriée. Plus spécifiquement, le bras droit de l'utilisateur touche son torse et crée un chemin électrique de plus faible résistance. Le courant i2, à la fréquence F2 plus élevée que la fréquence F1 du courant i1, emprunte cet autre chemin électrique et court-circuit partiellement le torse et le bras droit. La courant i1, du fait de sa fréquence F1, ne voit pas le court-circuit et emprunte le chemin au travers du membre et du tronc. Le dispositif de mesure calcule une impédance Z1b avec le courant i1 et une impédance Z2 avec le courant i2, mais du fait du court-circuit partiel, la valeur calculée de Z2b est plus basse que celle de Z2a.

Les Figures 8(c) et 8(d) illustrent une situation similaire, avec un court-circuit crée par un contact entre les jambes. En Figure 8(c), le dispositif de mesure injecte successivement deux courants i1 (à la fréquence F1), i2 (à la fréquence F2). On remarque ainsi que le courant i1 à la fréquence F1 ne permet pas de détecter une posture non adaptée, mais le courant i2 à la fréquence F2 le permet en mesurant une impédance Z2b qui diffère de l'impédance attendue Z2a.

Le dispositif de mesure 100, 200, 300, 400 est configuré pour comparer les impédances mesurées.

L'impédance d'un dipôle linéaire passif de bornes A et B (le corps humain et ses segments sont électriquement considérés comme des dipôles linéaires passifs) en régime sinusoïdal de courant et de tension est défini comme le quotient de la tension entre ses bornes et du courant qui le traverse. L'impédance est donc un nombre complexe comprenant un module et une phase. La partie réelle de l'impédance est appelée la résistance et la partie imaginaire de l'impédance est appelée la réactance.

Par "comparer les impédances mesurées", il est entendu comparer deux termes entre eux qui sont fonctions respectivement de chaque impédance mesurée. Par exemple, dans un mode de réalisation, la comparaison des impédances est une comparaison des modules d'impédances uniquement. La mesure du module d'impédance est en effet une mesure assez commode, notamment en ce qu'il est utilisé pour la détermination de la composition corporelle.

Toutefois, en variante, la comparaison des impédances peut être une comparaison des résistances des impédances uniquement. En variante, la comparaison des impédances est une comparaison uniquement des réactances des impédances. En variante, la comparaison des impédances est une comparaison fonction des phases des impédances. En variante, la comparaison des impédances est une comparaison fonction des modules, des résistances, des réactances et/ou des phases des impédances.

Par la suite de la description, il sera présenté plus en détail un mode de réalisation particulier de la comparaison de modules d'impédances.

Dans un mode de réalisation, le dispositif de mesure 100, 200, 300, 400 est configuré pour comparer l'impédance Z1 mesurée à la première fréquence F1 et l'impédance Z2 mesurée à la deuxième fréquence F2. Pour rappel, la fréquence F1 est une basse fréquence (par exemple 5kHz) et la fréquence Z2 est une haute fréquence (par exemple 250kHz).

Dans le mode de réalisation dans lequel le dispositif de mesure 100, 200, 300, 400 effectue des mesures d'impédance à au moins trois fréquences, le dispositif de mesure 100, 200, 300, 400, 500 est configuré en outre pour comparer l'impédance Z1 et l'impédance Z3 mesurée à la troisième fréquence F3 de courant, et/ou pour comparer l'impédance Z2 à l'impédance Z3.

Dans un mode de réalisation, le dispositif de mesure 100, 200, 300, 400, 500 est configuré pour comparer les impédances au niveau d'un même segment. Autrement dit, le dispositif de mesure 100, 200, 300, 400, 500 est configuré pour comparer les impédances d'un même segment, par exemple une impédance Z1 du bras gauche avec une impédance Z2 du bras gauche.

En particulier, le dispositif de mesure 100, 200, 300, 400, 500 est configuré pour comparer les impédances mesurées pour chaque segment analysé.

### Données d'impédance

Dans un mode de réalisation, la donnée d'impédance mesurée est une combinaison des impédances Z1 et Z2. Par exemple, la combinaison est une normalisation de l'impédance Z2 par l'impédance Z2, ce qui se traduit notamment par un ratio Z2/Z1 ou une différence normalisée (Z2-Z1)/Z1. Les inverses de ces formulations sont aussi possibles. L'intérêt de normer par l'impédance Z1, notamment en divisant, réside dans le fait que l'impédance Z1 varie peu en cas de posture non adaptée (contact peau-peau ou autre). Par conséquent, la division de Z2 par Z1 ne change pas le comportement de Z2 mais permet de normaliser sa valeur en fonction de chaque utilisateur par une valeur d'impédance de base. La ratio Z2/Z1 (ou Z3/Z1) reflète le comportement capacitif du corps humain ; or il a été vu qu'un contact peau-peau est équivalent à l'ajout de capacités dans le corps humain. La Figure 9 présente des résultats de données d'impédance normalisée par un ratio Z2/Z1 et Z3/Z1.

La normalisation permet en outre d'éviter une calibration sur l'utilisateur lui-même. En revanche, la normalisation nécessite toute même une pré-calibration, notamment à l'aide de mesures de test obtenus sur un ou plusieurs utilisateurs.

Dans un mode de réalisation, la donnée d'impédance est l'impédance mesurée elle-même.

### Donnée de référence

Pour déterminer si la mesure d'impédance est acceptable ou non, le dispositif de mesure compare la donnée d'impédance mesurée avec une donnée de référence. Selon les modes de réalisation, la donnée de référence peut être une donnée déterminée à partir d'impédance préalablement mesurée sur un ensemble d'utilisateur, ou bien une donnée calculée à partir du même utilisateur, soit préalablement, soit concomitamment.

La donnée de référence peut notamment être stockée par le dispositif de mesure afin d'être utilisée directement lors d'une mesure de composition corporelle.

### Seuil d'erreur

Dans un mode de réalisation, la comparaison est la comparaison de la donnée d'impédance (par exemple du ratio Z2/Z1 entre l'impédance Z1 à la première fréquence de courant F1 et l'impédance Z2 à la deuxième fréquence de courant F2) avec au moins une valeur seuil d'erreur Se1, Se2 (qui est une donnée de référence). En fonction de cette comparaison, un signal d'erreur peut être généré. Ce signal d'erreur peut être associé à une probabilité, afin de déterminer une probabilité que la mesure d'impédancemétrie soit acceptable ou non pour le dispositif de mesure.

En particulier, lorsque le ratio Z2/Z1 est inférieur à une première valeur seuil d'erreur Se1, alors le dispositif de mesure 100, 200, 300, 400, 500 est configuré pour générer un signal d'erreur. Ce signal d'erreur peut être significatif d'une posture inappropriée de l'utilisateur. Le signal d'erreur être notamment accompagné d'une probabilité associée, par exemple en fonction de la distance du ratio Z2/Z1 à la première valeur seuil d'erreur Se1.

Dans un mode de réalisation, lorsque le ratio Z2/Z1 est supérieur à une deuxième valeur seuil d'erreur Se2, alors le dispositif de mesure 100, 200, 300, 400, 500 est configuré pour générer un signal d'erreur. Le signal d'erreur peut être accompagné d'une probabilité associée, par exemple en fonction de la distance du ratio Z2/Z1 à la deuxième valeur seuil d'erreur SE2. En effet, une posture non-appropriée peut aussi générer des impédances anormalement élevées. Comme le ratio Z2/Z1 reflète le comportement capacitif du segment du corps mesuré, discriminer sur la base d'un ratio Z2/Z1 est pertinent pour rejeter une mesure d'impédance.

D'autres moyens de comparaison entre l'impédance Z1 à la première fréquence F1 et l'impédance Z2 à la deuxième fréquence F2 qu'un ratio sont possibles.

### Seuil d'incertitude

Dans un mode de réalisation, le dispositif de mesure 100, 200, 300, 400, 500 est configuré pour effectuer une comparaison additionnelle du ratio Z2/Z1 entre l'impédance Z1 à la première fréquence F1 et l'impédance Z2 à la deuxième fréquence F2 avec au moins une valeur seuil d'incertitude Si1, Si2 (qui sont des données de référence). Le principe est identique à celui du seuil d'erreur sauf que la valeur du seuil est modifiée. En particulier, la première valeur seuil d'incertitude Si1 est notamment plus élevée que la première valeur seuil d'erreur Se1, de sorte que le seuil d'incertitude concerne davantage de mesures.

Lorsque le ratio Z2/Z1 est inférieur à une première valeur seuil d'incertitude SI1, le dispositif de mesure 100, 200, 300, 400, 500 est configuré pour générer un signal d'alerte. Ce signal d'alerte peut être significatif d'une incertitude sur la posture de l'utilisateur. Le signal d'alerte peut être accompagné d'une probabilité associée, par exemple en fonction de la distance du ratio Z2/Z1 à la première valeur seuil d'incertitude Si1.

Dans un mode de réalisation, de façon similaire au deuxième seuil d'erreur Se2, lorsque le ratio Z2/Z1 est supérieur à une deuxième valeur seuil d'incertitude Si2 alors le dispositif de mesure 100, 200, 300, 400, 500 est configuré pour générer également un signal d'alerte. Le signal d'alerte peut être accompagné d'une probabilité associée, par exemple en fonction de la distance du ratio Z2/Z1 à la deuxième valeur seuil d'incertitude SI2.

La deuxième valeur seuil d'incertitude SI2 est notamment moins élevée que la deuxième valeur seuil d'erreur Se2.

Dans la configuration présentée, pour un seuil qui concerne les ratios Z2/Z1, on a donc Se1<Si1<Si2<Se2.

### Utilisation de la fréquence F3

Dans un mode de réalisation avantageux, la comparaison comprend en outre la comparaison d'un ratio Z3/Z1 entre l'impédance Z1 à la première fréquence F1 et l'impédance Z3 à la deuxième fréquence F3 avec une première valeur seuil d'erreur additionnelle Se1'. Lorsque le ratio Z3/Z1 est inférieur à la première valeur seuil d'erreur additionnelle Se1', alors le dispositif de mesure 100, 200, 300, 400, 500 est configuré pour générer un signal d'erreur. Ce signal d'erreur peut être significatif d'une posture inappropriée de l'utilisateur. Le signal d'erreur peut être accompagné d'une probabilité associée, par exemple en fonction de la distance du ratio Z3/Z1 à la première valeur seuil d'erreur additionnelle Se1'.

Dans un mode de réalisation, lorsque le ratio Z3/Z1 est supérieur à une deuxième valeur seuil additionnelle Se2', le dispositif de mesure 100, 200, 300, 400, 500 est configuré pour générer un signal d'erreur. Le signal d'erreur peut être accompagné d'une probabilité associée, par exemple en fonction de la distance du ratio Z2/Z1 à la deuxième valeur seuil d'erreur additionnelle SE2'.

Dans un mode de réalisation avantageux, le dispositif de mesure 100, 200, 300, 400, 500 est configuré pour comparer un ratio Z3/Z1 entre l'impédance Z1 à la première fréquence F1 et l'impédance Z3 à la deuxième fréquence F3 avec au moins une valeur seuil d'incertitude additionnelle Si1', Si2'.

En particulier, lorsque le ratio Z3/Z1 est inférieur à une première valeur seuil d'incertitude additionnelle SI1', alors le dispositif de mesure 100, 200, 300, 400, 500 est configuré pour générer un signal d'alerte, notamment lié à une incertitude sur la posture de l'utilisateur. Le signal d'alerte peut être accompagné d'une probabilité associée, par exemple en fonction de la distance du ratio Z3/Z1 à la première valeur seuil d'incertitude additionnelle SI1'.

Dans un mode de réalisation avantageux, lorsque le ratio Z3/Z1 est supérieur à une deuxième valeur seuil d'incertitude additionnelle SI2', alors le dispositif de mesure 100, 200, 300, 400, 500 est configuré pour notamment générer un signal d'erreur. Le signal d'alerte est notamment accompagné d'une probabilité associée, par exemple en fonction de la distance du ratio Z3/Z1 à la deuxième valeur seuil d'incertitude additionnelle SI2'.

### Détermination du seuil d'erreur

Dans un mode de réalisation, chaque valeur seuil d'erreur Se1, Se2 est un seuil d'erreur prédéterminé.

En particulier, chaque seuil prédéterminé Se1, Se2 peut être obtenu par analyse d'une pluralité de mesures d'impédance préalables sur plusieurs utilisateurs. Ainsi, le seuil prédéterminée Se1, Se2 se détermine en utilisant les ratios Z2/Z1 de plusieurs utilisateurs.

Dans un mode de réalisation, chaque seuil d'erreur prédéterminé Se1, Se2 est identique pour tout utilisateur.

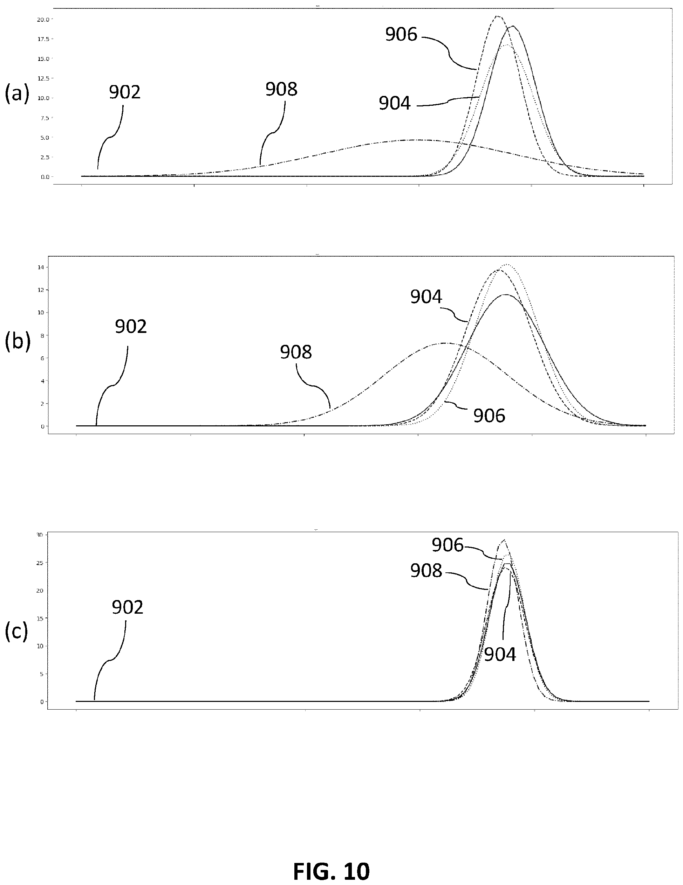

Les Figures 9 et 10 représentent les résultats d'une campagne de mesure faite sur 34 utilisateurs avec le dispositif de mesure selon le mode de réalisation de la Figure 3.

La Figure 9 représente la distribution du ratio des modules d'impédance Z2/Z1 avec la première fréquence F1 à 5 kHz et la deuxième fréquence F2 à 250 kHz, respectivement dans le bras gauche (a), dans l'hémicorps gauche (b) et dans la jambe gauche (c), dans les cas suivants :
- mesure en étant habillé et ainsi pas de risque de contact de peaux entre les bras et le tronc et/ou les jambes (courbes 902 en trait plein),
- mesure en sous-vêtements sans consigne précise pour l'utilisateur (courbes 904 en pointillé large régulier),
- mesure en sous-vêtements avec une consigne pour l'utilisateur d'écarter les bras et les jambes (courbes 906 en pointillé court régulier),
- mesure en sous-vêtements avec une consigne pour l'utilisateur de mettre les bras le long du corps et donc de provoquer un contact de peaux entre les bras et le tronc et/ou la jambe (courbes 908 en pointillé alternant).

Les résultats montrent un ratio moyen Z2/Z1 dans les cas "habillé" et "sous-vêtements consigne" présentant une faible dispersion des valeurs. Dans le cas "sous-vêtements normal", on note une valeur moyenne un peu plus basse et une dispersion des valeurs un peu plus élevée. Cela traduit le fait que certains utilisateurs adoptent une posture inappropriée de mesure lorsqu'ils n'ont pas reçu de consigne. Enfin, dans le cas "sous-vêtements bras", les valeurs sont très dispersées avec une moyenne plus basse encore. La dispersion est particulièrement élevée dans les bras et l'hémicorps. Cela confirme qu'une valeur faible du ratio Z2/Z1 est corrélée avec une probabilité élevée de posture inappropriée de l'utilisateur.

La Figure 10 représente la distribution du ratio des valeurs des modules d'impédance Z2/Z1 avec la première fréquence F1 à 5 kHz et la deuxième fréquence F2 à 50 kHz, respectivement dans le bras gauche, dans l'hémicorps gauche et dans la jambe gauche, dans les cas suivants :
- mesure en étant habillé et ainsi pas de risque de contact de peaux entre les bras et le tronc et/ou les jambes (courbes 902 en trait plein),
- mesure en sous vêtement sans consigne précise pour l'utilisateur (courbes 904 en pointillé large régulier),
- mesure en sous vêtement avec une consigne pour l'utilisateur d'écarter les bras et les jambes (courbes 906 en pointillé court régulier),
- mesure en sous-vêtements avec une consigne pour l'utilisateur de mettre les bras le long du corps et donc de provoquer un contact de peaux entre les bras et le tronc et/ou la jambe (courbes 908 en pointillé alternant).

Les résultats sont comparables à ceux de la Figure 9. Toutefois, la chute d'impédance en cas de court-circuit partiel est moins élevée du fait d'une deuxième fréquence F2 plus faible que dans le cas précédent.

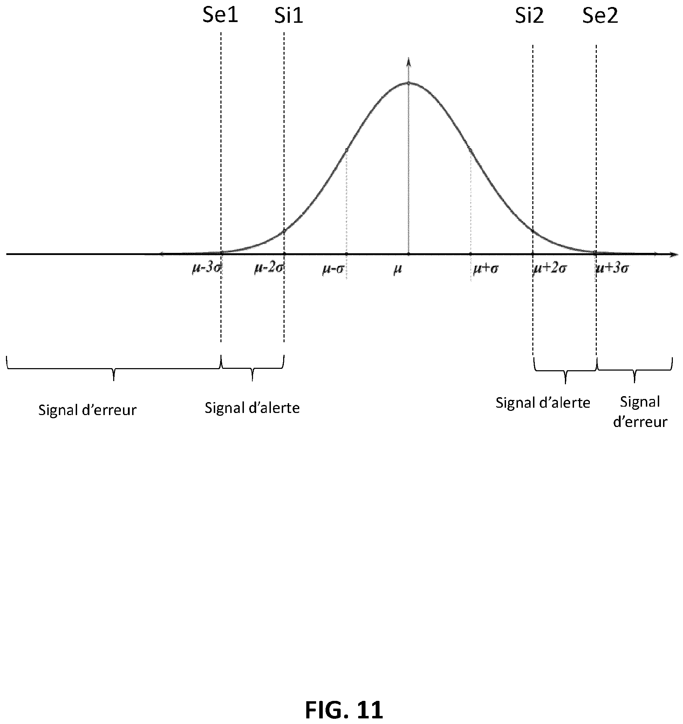

En référence à la Figure 11 qui présente une distribution de ratio d'impédances avec deux valeurs seuil d'erreur et deux valeurs seuil d'incertitude, le premier seuil d'erreur prédéterminé Se1 peut être choisi de manière statistique, par exemple à une valeur égale à trois fois l'écart-type de la distribution en dessous de la valeur moyenne de la distribution dans le cas de référence (ici le cas "habillé").

Le deuxième seuil d'erreur prédéterminé Se2 peut être choisi de manière statistique, par exemple à une valeur égale à trois fois l'écart-type de la distribution au-dessus de la valeur moyenne de la distribution dans le cas de référence (ici le cas "habillé").

Dans un mode de réalisation, chaque seuil d'erreur prédéterminé Se1, Se2 est fonction du sexe de l'utilisateur.

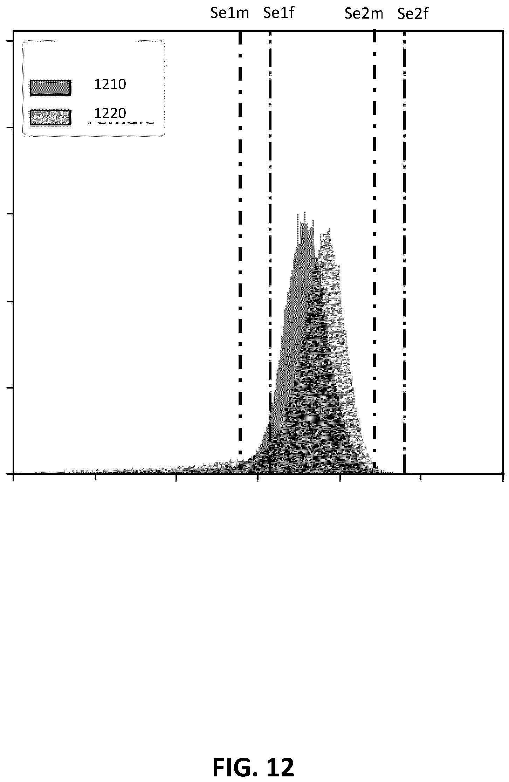

La Figure 12 représente la distribution de ratio d'impédance à 250 kHz et 5 kHz (i.e. le ratio Z(250kHz)/Z(5kHz)) dans le corps complet pour les hommes (en gris foncé 1210) et pour les femmes (en gris clair 1220). On remarque un décalage des gaussiennes associées à chaque sexe.

Ainsi, il peut être défini des seuils d'erreur prédéterminé pour les hommes Se1m, Se2m et des seuils d'erreur prédéterminé pour les femmes Se1f, Se2f.

En variante ou en option, chaque seuil d'erreur prédéterminé Se1, Se2 est fonction du poids de l'utilisateur, notamment fonction de l'Indice de Masse Corporelle (IMC).

En variante ou en option, chaque seuil d'erreur prédéterminé Se1, Se2 est fonction de la composition corporelle de l'utilisateur, notamment du pourcentage de masse grasse de l'utilisateur.

Dans un mode de réalisation, chaque valeur seuil d'incertitude Si1, Si2 est un seuil d'incertitude prédéterminé.

En référence à la Figure 11, le seuil d'incertitude prédéterminé Si1 peut être choisi de manière statistique, par exemple à une valeur égale à deux fois l'écart-type de la distribution en dessous de la valeur moyenne de la distribution dans le cas de référence (ici le cas "habillé").

Le deuxième seuil d'incertitude prédéterminé Si2 peut être choisi de manière statistique, par exemple à une valeur égale à deux fois l'écart-type de la distribution au-dessus de la valeur moyenne de la distribution dans le cas de référence (ici le cas "habillé").

Dans un mode de réalisation avantageux, chaque seuil d'incertitude prédéterminé Si1, Si2 est fonction du sexe de l'utilisateur.

Il peut être défini des seuils d'incertitude prédéterminé pour les hommes Si1m, Si2m et des seuils d'incertitudes prédéterminé pour les femmes Si1f, Si2f.

En variante ou en option, chaque seuil d'incertitude prédéterminé Si1, Si2 est fonction du poids de l'utilisateur, notamment fonction de l'Indice de Masse Corporelle (IMC).

En variante ou en option, chaque seuil d'incertitude prédéterminé Si1, SI2 est fonction de la composition corporelle de l'utilisateur, notamment du pourcentage de masse grasse de l'utilisateur.

### Comparaison avec un ratio de référence propre à l'utilisateur

Dans un mode de réalisation, chaque valeur seuil d'erreur Se1, Se2 est propre à l'utilisateur, notamment fonction d'un ratio d'impédances de référence propre à l'utilisateur.

En particulier, le ratio d'impédances de référence est un ratio entre une impédance Z1 de référence mesurée à la première fréquence F1 et une impédance Z2 de référence mesurée à la deuxième fréquence F2 mesurées préalablement sur l'utilisateur. Les mesures préalables de l'impédance Z1 de référence et de l'impédance Z2 sont préférentiellement faites de manière guidée et/ou avec des consignes à l'utilisateur permettant une probabilité élevée de bonne posture de mesure.

La première valeur seuil d'erreur Se1 est par exemple égale à un pourcentage du ratio d'impédances de référence, notamment 80% du ratio d'impédances de référence. La deuxième valeur seuil d'erreur Se2 est par exemple égale à un pourcentage du ratio d'impédances de référence, notamment 120% du ratio d'impédances de référence.

Dans un mode de réalisation, chaque valeur seuil d'incertitude Si1, Si2 est fonction du ratio d'impédances de référence propre à l'utilisateur.

La première valeur seuil d'incertitude Si1 est par exemple égale à un pourcentage du ratio d'impédances de référence, notamment 90% du ratio d'impédances de référence. La deuxième valeur seuil d'incertitude Si2 est par exemple égale à un pourcentage du ratio d'impédances de référence, notamment 110% du ratio d'impédances de référence.

### Comparaison avec l'historique de l'utilisateur

Dans un mode de réalisation, chaque valeur seuil d'erreur Se1, Se2 est déterminée en fonction d'un historique de ratio d'impédances propre à l'utilisateur.

En particulier, l'historique de ratio d'impédances est une moyenne d'au moins deux ratio Z2/Z1 entre une impédance Z1 à la première fréquence F1 et une impédance Z2 à la deuxième fréquence F2 mesurées précédemment sur l'utilisateur.

La première valeur seuil d'erreur Se1 est par exemple égale à un pourcentage de la moyenne des ratios Z2/Z1 de l'historique de l'utilisateur, notamment 80% du ratio d'impédances moyen. La deuxième valeur seuil d'erreur Se2 est par exemple égale à un pourcentage de la moyenne des ratios Z2/Z1 de l'historique de l'utilisateur, notamment 120% du ratio d'impédances moyen.

Dans un mode de réalisation, chaque valeur seuil d'incertitude Si1, Si2 est déterminée en fonction d'un historique de ratio d'impédances propre à l'utilisateur.

La première valeur seuil d'incertitude Si1 est par exemple égale à un pourcentage de la moyenne des ratios Z2/Z1 de l'historique de l'utilisateur, notamment 90% du ratio d'impédances moyen. La deuxième valeur seuil d'incertitude Si2 est par exemple égale à un pourcentage de la moyenne des ratios Z2/Z1 de l'historique de l'utilisateur, notamment 110% du ratio d'impédances moyen.

### Variantes

Le mode de réalisation précédent (comparaison avec l'historique de l'utilisateur) peut par ailleurs être mis en oeuvre avec différentes variantes.

Dans un mode de réalisation, la donnée d'impédance comprend la mesure d'impédance Z2 uniquement (sans normalisation). Le choix de Z2 se justifie par le fait que les hautes-fréquences sont les fréquences les plus impactées par des courts-circuits. Dans ce cas, la valeur seuil d'erreur est calculée sur la même base, à savoir des valeurs de mesures d'impédance, sur un segment, dit segment de référence.

La fréquence utilisée ici est par exemple comprise entre 40 kHz et 5000 kHz, notamment 250 kHz.

Dans une variante, la comparaison peut s'effectuer sur un même segment : par exemple, la mesure d'impédance Z2 d'un segment est comparée à une valeur correspondant à un historique de mesure d'impédance Z2 du même segment (la donnée de référence). Le segment mesuré et le segment de référence sont identiques. Une marge peut être ajoutée à cette valeur d'historique, pour prendre en compte des variations de poids entre deux mesures espacées temporellement. Une mesure d'impédance Z1 à la fréquence F1 peut en outre être effectuée, pour servir de valeur d'impédance de base. La comparaison peut alors comprendre une variation d'impédance Z2 et une variation d'impédance Z1 et un signal d'erreur est généré lorsqu'une variation d'impédance Z2 est supérieur à une seuil d'erreur et que la variation d'impédance Z1 est inférieur à un seuil de base. Alternativement ou en complément, la marge peut être déterminée par une variation d'impédance mesurée à la fréquence Z1, pour tenir compte d'une évolution du corps de l'utilisateur.

Dans une autre variante, la comparaison s'effectue sur la valeur d'impédance d'un autre segment (la donnée de référence). Le segment mesuré et le segment de référence sont différents. En effet, au sein d'un même utilisateur, l'impédance des segments présente généralement des similarités. En cas de posture non appropriée, avec un court-circuit, l'impédance d'un segment risque de baisser. Par conséquent, en comparant des valeurs d'impédance au sein d'un même corps, le dispositif de mesure peut identifier une posture non appropriée. En particulier, l'autre segment peut être le symétrique du segment (segment de référence non identique mais similaire au segment mesuré) : par exemple, la mesure d'impédance d'un côté est comparée à une valeur d'impédance de l'autre côté. La symétrie du corps humain permet d'utiliser un tel critère. Dans cette variante, la donnée de référence peut être déterminée concomitamment à la donnée d'impédance, c'est-à-dire sur la même session de mesure. Alternativement, l'autre segment peut être un autre segment, quelconque, comme l'arc-de-jambe (segment de référence non identique et non similaire).

Les principes de seuil et d'alerte s'appliquent dans ces variantes de façon similaire.

### Méthode de mesure d'impédancemétrie

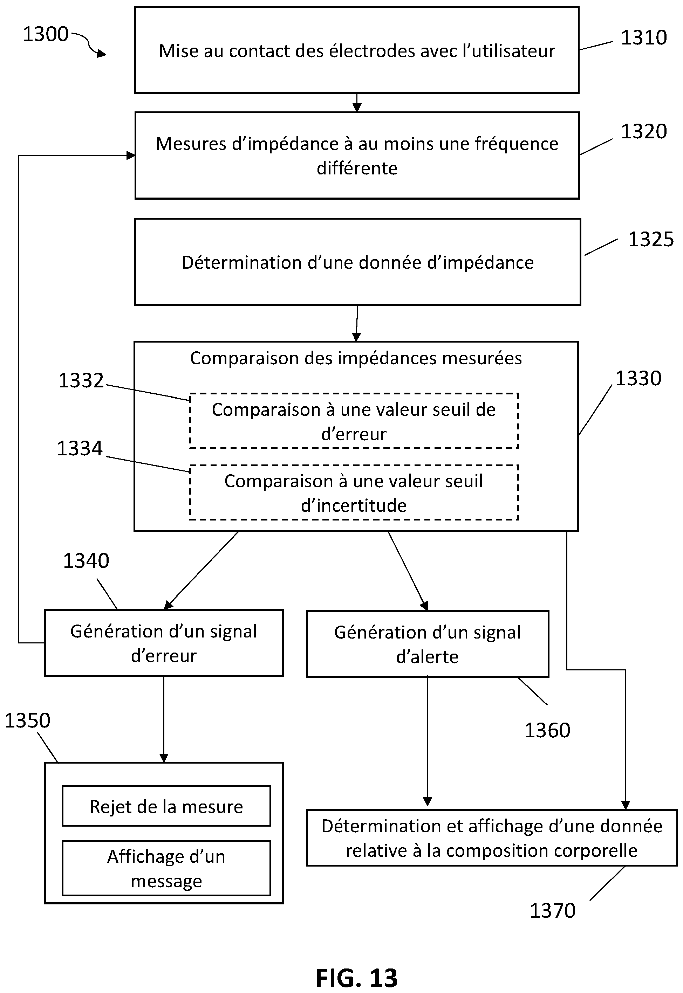

Une méthode 1300 de mesure d'impédance mise en oeuvre par le dispositif de mesure 100, 200, 300, 400, 500 va être décrite par la suite, en référence à la Figure 13. La méthode 1300 va être décrite en relation avec le dispositif de mesure de la figure 2 mais s'applique à tout dispositif de mesure 100, 200, 300, 400, 500 permettant de faire une analyse d'impédancemétrie segmentale. L'intérêt des dispositifs 200, 400 est qu'il permet d'analyser tous les segments avec une seule position.

Lors d'une étape initiale 1310, les électrodes sont mises en contact avec l'utilisateur. En particulier, les au moins deux électrodes d'injection 212, 214, 216, 218 et les au moins deux électrodes de mesure 222, 224, 226, 228 sont mises au contact des mains et/ou des pieds de l'utilisateur. Dans le mode de réalisation illustré sur la Figure 3, l'utilisateur monte avec ses pieds sur la base 302 du pèse-personne et saisit la poignée 304 avec ses mains.

Puis, lors d'une étape 1320, le dispositif de mesure 100, 200, 300, 400, 500 effectue des mesures d'impédance sur l'utilisateur à au moins une fréquence de courant alternatif, ou, selon des modes réalisation, au moins deux fréquences différentes de courant alternatif.

Dans une étape 1325, le dispositif de mesure 100, 200, 300, 400, 500 calcule une donnée d'impédance à partir des impédances mesurées, ainsi que cela a été décrit précédemment.

Puis, lors d'une étape 1330, le dispositif de mesure 100, 200, 300, 400, 500 compare les impédances mesurées à l'étape précédente 1320, notamment à l'aide de la donnée d'impédance. En particulier, dans un mode de réalisation, le dispositif de mesure 100, 200, 300, 400, 500 compare l'impédance Z1 mesurée à la première fréquence F1 et l'impédance Z2 mesurée à la deuxième fréquence F2.

Dans le mode de réalisation dans lequel le dispositif de mesure 100, 200, 300, 400, 500 effectue des mesures d'impédance à au moins trois fréquences, le dispositif de mesure 100, 200, 300, 400, 500 peut en outre comparer l'impédance Z1 et l'impédance Z3 mesurée à la troisième fréquence F3 de courant, et/ou comparer l'impédance Z2 à l'impédance Z3. La comparaison se fait par rapport à une donnée de référence, qui a été décrite précédemment (notamment une comparaison avec seuil d'erreur en étape 1332 et comparaison avec un seuil d'incertitude en étape 1334). Le type de donnée de référence et de comparaison dépend ainsi de la donnée d'impédance.

Les différentes comparaisons possibles d'impédances ont été décrites en détail ci-dessus et ne seront pas reprises ici par soucis de concision, notamment le mode de réalisation utilisant le ratio d'impédance Z2/Z1.

Puis, lors d'une étape 1340, le dispositif de mesure 100, 200, 300, 400, 500 génère un signal d'erreur lors des mesures d'impédances sur la base de la comparaison de l'impédance Z1 à la première fréquence F1 et de l'impédance Z2 à la deuxième fréquence F2. En particulier, le dispositif de mesure 100, 200, 300, 400, 500 génère un signal d'erreur de l'utilisateur lorsque le ratio Z2/Z1 est inférieur à la valeur seuil d'erreur Si1 ou supérieur à la deuxième valeur seuil d'erreur Si2.

Le signal d'erreur est par exemple lié à une détection d'une posture inappropriée de l'utilisateur, notamment un contact de peau entre deux parties du corps de l'utilisateur.

Puis, lors d'une étape 1350, le dispositif de mesure 100, 200, 300, 400, 500 peut rejeter les mesures d'impédance en réponse à la génération du signal d'erreur. Autrement dit, le dispositif de mesure 100, 200, 300, 400, 500 ne transmet pas ces mesures aux dispositifs tiers, notamment au serveur 520 et/ou au terminal mobile 530, via le réseau de communication 510 ou alors il transmet ces mesures aux dispositifs tiers en leur attachant un tag d'erreur (appelé « flag »). En variante ou en option, lors de l'étape 1350, le dispositif de mesure 100, 200, 300, 400, 500 affiche un message à destination de l'utilisateur en cas de génération du signal d'erreur, par exemple au moyen de l'affichage 308, 408. Le message peut comprendre une information indiquant qu'une erreur a été détectée, et/ou indiquant une posture inappropriée, et par exemple rappeler les recommandations de bonne posture à l'utilisateur. Le message peut également indiquer sur quels segments une suspicion de contact a été détectée, par exemple un contact entre le bras gauche et la jambe gauche ou un contact entre les deux jambes.

En complément ou variante, le dispositif de mesure 100, 200, 300, 400, 500 peut envoyer les mesures d'impédance, ou de composition corporelle associées, aux dispositifs tiers, notamment au serveur 520 et/ou au terminal mobile 530, avec une information indiquant qu'une erreur a été détectée (par exemple par l'ajout d'un tag à la mesure). Les dispositifs tiers peuvent alors rejeter au moins une partie des mesures ou les garder. Dans un mode de réalisation, les dispositifs tiers peuvent afficher un message à destination de l'utilisateur en cas d'erreur, par exemple via l'écran du terminal mobile 530. Le message peut comprendre une information indiquant qu'une erreur a été détectée et par exemple rappeler les recommandations de bonne posture à l'utilisateur.

Dans un mode de réalisation dans lequel une pluralité de mesures d'impédances sont effectuées pour plusieurs segments, le dispositif de mesure 100, 200, 300, 400, 500 peut rejeter uniquement les mesures d'impédance affectées par l'erreur et accepter les mesures d'impédance non affectées. En effet, le dispositif de mesure est capable de générer une erreur segment par segment (bras gauche, bras droit, jambe gauche, jambe droite, côté gauche, côté droit, arc de bras, arc de jambe, tronc). Par exemple, si un contact entre le bras gauche et la jambe gauche est détecté via la détection d'un ratio d'impédances inférieur à la valeur seuil d'erreur SE1 sur le segment "bras gauche" mais qu'aucun contact n'est détecté dans les membres du côté droit, seules les mesures pour les segments de gauche sont rejetées.

Dans un mode de réalisation, le dispositif de mesure 100, 200, 300, 400, 500 peut générer de nouvelles mesures d'impédance en cas de génération du signal d'erreur et ainsi revenir à l'étape 1320.

Dans une étape 1360, alternative à l'étape 1350, le dispositif de mesure 100, 200, 300, 400, 500 génère un signal d'alerte, notamment lié à une incertitude de posture de l'utilisateur lors des mesures d'impédance, sur la base de la comparaison de l'impédance Z1 à la première fréquence F1 et de l'impédance Z2 à la deuxième fréquence F2. En particulier, comme illustré sur la Figure 11, le dispositif de mesure 100, 200, 300, 400, 500 génère un signal d'alerte lorsque le ratio Z2/Z1 entre l'impédance Z1 à la première fréquence F1 et l'impédance Z2 à la deuxième fréquence F2 est compris entre la valeur seuil d'erreur Se1 et la valeur seuil d'incertitude Si1. En variante ou en complément, le dispositif de mesure 100, 200, 300, 400, 500 génère un signal d'alerte lorsque le ratio Z2/Z1 entre l'impédance Z1 à la première fréquence F1 et l'impédance Z2 à la deuxième fréquence F2 est compris entre la valeur seuil d'incertitude Si2 et la valeur seuil d'erreur Se2.

Puis, lors d'une étape 1370, le dispositif de mesure 100, 200, 300, 400, 500 peut déterminer au moins une donnée relative à la composition corporelle de l'utilisateur, par exemple le pourcentage de masse grasse et musculaire par segment, en fonction des mesures d'impédance aux au moins deux fréquences différentes. Le dispositif de mesure 100, 200, 300, 400, 500 peut afficher ces données sur l'affichage 308, 408 avec éventuellement un message d'incertitude. En variante ou en option, le dispositif de mesure 100, 200, 300, 400, 500 peut envoyer ces données aux dispositifs tiers, notamment au serveur 520 et/ou au terminal mobile 530, avec le signal d'alerte associé, et par exemple indiquant au niveau de quels segments l'incertitude se situe. L'alerte peut être affichée à l'utilisateur en même temps que la mesure, par exemple au moyen de l'affichage 308, 408 et/ou via le terminal mobile 530 afin notamment de lui rappeler les consignes de mesure.

Lorsqu'une erreur ou une incertitude est générée, autrement dit lorsque le ratio Z2/Z1 entre l'impédance Z1 à la première fréquence F1 et l'impédance Z2 à la deuxième fréquence F2 est supérieur à la première valeur seuil d'incertitude Si1, et par exemple inférieur à la deuxième valeur d'incertitude SI2, le dispositif de mesure 100, 200, 300, 400, 500 met en oeuvre directement l'étape 1370 et détermine au moins une donnée relative à la composition corporelle de l'utilisateur.

### Exemples de mise en œuvre

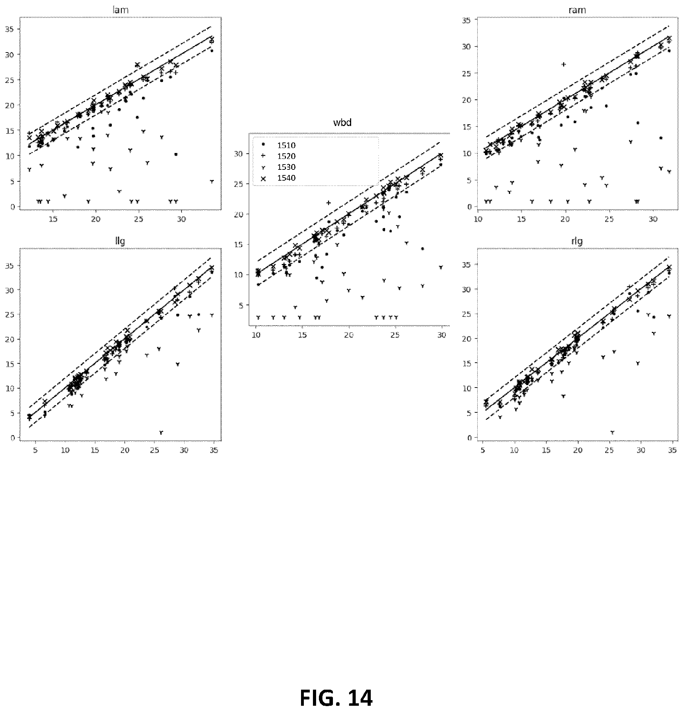

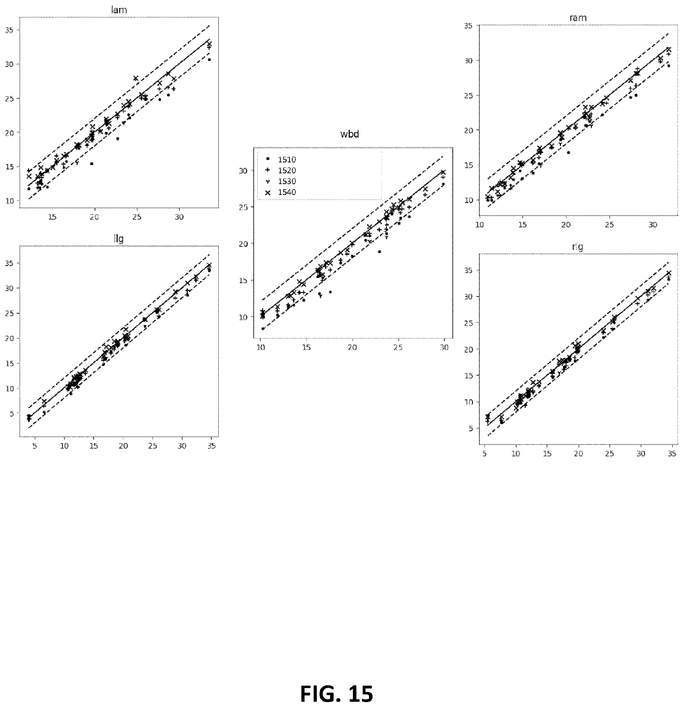

Les Figures 14 et 15 représentent les pourcentages de masse grasse obtenus à partir de la même campagne de mesure que les résultats illustrés sur les Figures 9 et 10, sans détection d'erreur pour la Figure 14 et avec détection et rejet des mesures liées à des erreurs pour la Figure 15.

Les Figures 14 et 15 représentent de gauche à droite et de haut en bas, les pourcentages de masse grasse déterminés en fonction du pourcentage de masse grasse obtenu dans le cas de référence "habillé" pour le bras gauche (lam), le torse (torso), le bras droit (ram), la jambe gauche (Ilg), le corps complet (wbd) et la jambe droite (rlg). Théoriquement, si la répétabilité des mesures étaient parfaites, toutes les mesures devraient se retrouver sur la droite identité y=x. A l'inverse, toute mesure s'écartant fortement de cette droite identité y=x indique un calcul du pourcentage de masse grasse perturbé par un facteur de position, notamment un court-circuit, provoqué par une posture non adaptée à la mesure.

De manière similaire aux Figures 9 et 10, on remarque sur la Figure 14 que dans les cas "sous-vêtements consigne" (représenté par des + 1520) les valeurs semblent cohérentes avec la référence "habillé" (représenté par des x 1540). Dans le cas "sous-vêtements normal" (représenté par des points 1510), on note certaines valeurs problématiques. Enfin, dans le cas "sous-vêtements bras" (représenté par des étoiles à trois branches 1530), les valeurs sont très dispersées et éloignées des valeurs de référence "habillé".

La Figure 15 présentent les résultats à la suite de la mise en oeuvre de la détection d'erreurs telle que décrite ci-dessus et le rejet des mesures problématiques avec une première valeur seuil d'erreur SE1 prédéterminée égale à trois fois l'écart-type de la distribution en dessous de la valeur moyenne du cas de référence et une deuxième valeur seuil d'erreur SE2 prédéterminée égale à trois fois l'écart-type de la distribution au-dessus de la valeur moyenne du cas de référence. La Figure 15 montre une cohérence des valeurs de pourcentage de masse grasse fortement améliorée et un rejet avec succès des valeurs les plus problématiques. La détermination du pourcentage de masse grasse de l'utilisateur est ainsi fiabilisée et plus reproductible.

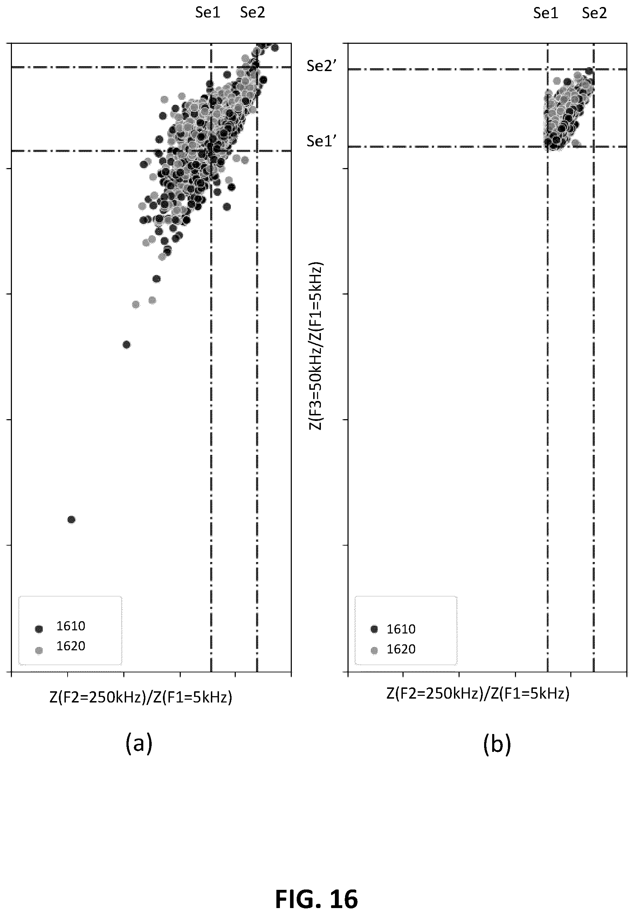

La Figure 16 représente pour une pluralité de mesures effectuées avec le dispositif de mesure de la Figure 3, les ratios d'impédance Z2/Z1 en abscisse et Z3/Z1 en ordonné avec Z1=5 kHz, Z3=50 kHz et Z2=250 kHz. La Figure 16 représente les valeurs associées au genre masculin (points noir 1610) et au genre féminin (points gris 1620). Le graphique (a) représente les mesures dans le bras gauche sans détection d'erreur et le graphique (b) représente les mesures dans corps entier selon la méthode 1300 avec rejet des mesures pour lequel un signal d'erreur a été généré. Les premières et deuxièmes valeurs seuil d'incertitude Se1, Se2 et les premières et deuxièmes valeurs seuil d'incertitude additionnelle Se1', Se2' sont représentées sur les graphiques. Du fait de la génération du signal d'erreur à l'aide des intervalles [Se1, Se2], [Se1', Se2'], on remarque sur le graphique (a) que plusieurs mesures sont en dehors d'au moins l'un des intervalles [Se1, Se2], [Se1', Se2'] et sont donc potentiellement problématiques. A l'inverse, on remarque sur le graphique (b) que toutes les mesures sont bien comprises dans les deux intervalles [Se1, Se2] et [Se1', Se2'] et que la détection d'erreur a rejeté les mesures potentiellement problématiques, rendant la mesure du pourcentage de masse grasse de l'utilisateur plus fiable et plus reproductible.

## Revendications

1. Dispositif de mesure (100, 200, 300, 400, 500) de composition corporelle configuré pour :
- effectuer des mesures d'impédance sur un utilisateur (U) à au moins deux fréquences différentes (F1, F2, F3), dont une première fréquence (F1) et une deuxième fréquence (F2) plus élevée que la première fréquence (F1),
- comparer l'impédance (Z1) mesurée à la première fréquence (F1) et l'impédance (Z2) mesurée à la deuxième fréquence (F2),
- générer un signal d'erreur sur la base de la comparaison de l'impédance (Z1) mesurée à la première fréquence (F1) et de l'impédance (Z2) mesurée à la deuxième fréquence (F2).

2. Dispositif de mesure (100, 200, 300, 400, 500) selon la revendication 1, configuré pour :
- en réponse à l'absence de génération de signal d'erreur, déterminer au moins une donnée de composition corporelle en en fonction de l'impédance (Z1) à la première fréquence (F1) et de l'impédance (Z2) à la deuxième fréquence (F2).

3. Dispositif de mesure (100, 200, 300, 400, 500) selon la revendication 1 ou 2, configuré pour:
- générer une notification pour l'utilisateur, la notification portant sur une erreur et/ou une posture inappropriée pour l'utilisateur, sur la base du signal d'erreur, notamment un contact de peau entre deux parties du corps de l'utilisateur (U).

4. Dispositif de mesure (100, 200, 300, 400, 500) selon l'une quelconque des revendications précédentes, dans lequel le dispositif de mesure (100, 200, 300, 400, 500) est configuré pour comparer l'impédance (Z1) mesurée à la première fréquence (F1) et l'impédance (Z2) mesurée à la deuxième fréquence (F2) au niveau d'un même segment de l'utilisateur.

5. Dispositif de mesure (100, 200, 300, 400, 500) selon l'une quelconque des revendications précédentes, dans lequel le dispositif de mesure (100, 200, 300, 400, 500) est configuré, en réponse à la génération d'un signal d'erreur, pour :
- rejeter la mesure d'impédance, et/ou
- afficher un message à destination de l'utilisateur, et/ou
- relancer une nouvelle mesure d'impédance.

6. Dispositif de mesure (100, 200, 300, 400, 500) selon l'une quelconque des revendications précédentes, dans lequel la comparaison est la comparaison d'un ratio (Z2/Z1) entre l'impédance (Z2) mesurée à la première fréquence (F1) et l'impédance (Z2) mesurée à la deuxième fréquence (F2) avec au moins une valeur seuil d'erreur (Se1, Se2).

7. Dispositif de mesure (100, 200, 300, 400, 500) selon la revendication 6, dans lequel la ou chaque valeur seuil d'erreur (Se1, Se2) est un seuil d'erreur prédéterminé, par exemple obtenu par analyse d'une pluralité de mesures d'impédance préalables sur plusieurs utilisateurs.

8. Dispositif de mesure (100, 200, 300, 400, 500) selon la revendication 6, dans lequel la ou chaque valeur seuil d'erreur (Se1, Se2) est propre à l'utilisateur, en particulier fonction d'un ratio d'impédances de référence propre à l'utilisateur, le ratio d'impédances de référence étant par exemple un ratio entre une impédance de référence à la première fréquence et une impédance de référence à la deuxième fréquence mesurées préalablement sur l'utilisateur.

9. Dispositif de mesure (100, 200, 300, 400, 500) selon la revendication 6, dans lequel la ou chaque valeur seuil d'erreur (Se1, Se2) est déterminée en fonction d'un historique de ratio d'impédances propre à l'utilisateur, l'historique de ratio d'impédances étant par une moyenne d'au moins deux ratios entre une impédance à la première fréquence et une impédance à la deuxième fréquence mesurées précédemment sur l'utilisateur.

10. Dispositif de mesure (100, 200, 300, 400, 500) selon l'une quelconque des revendications 6 à 9, dans laquelle la comparaison comprend une comparaison additionnelle du ratio entre l'impédance (Z1) à la première fréquence (F1) et l'impédance (Z2) à la deuxième fréquence (F2) avec au moins une valeur seuil d'incertitude (Si1, Si2), et dans lequel le dispositif de mesure (100, 200, 300, 400) est configuré pour générer un signal d'alerte sur la base de la comparaison additionnelle.

11. Dispositif de mesure (100, 200, 300, 400, 500) selon l'une quelconque des revendications précédentes, configuré pour :
- effectuer une mesure d'impédance (Z1, Z2, Z3) à au moins trois fréquences, dont la première fréquence (F1), la deuxième fréquence (F2) et une troisième fréquence (F3) comprise entre la première fréquence (F1) et la deuxième fréquence (F2),
- comparer l'impédance (Z1) mesurée à la première fréquence (F1) et l'impédance (Z2) mesurée à la deuxième fréquence (F2),
- comparer l'impédance (Z1) mesurée à la première fréquence (F1) et l'impédance (Z3) mesurée à la deuxième fréquence (F3),
- générer un signal d'erreur sur la base de la comparaison de l'impédance (Z1) à la première fréquence (F1) et de l'impédance (Z2) à la deuxième fréquence (F2) et la comparaison de l'impédance (Z1) à la première fréquence (F1) et de l'impédance (Z3) à la deuxième fréquence (F3).

12. Dispositif de mesure (100, 200, 300, 400, 500) selon l'une quelconque des revendications précédentes, dans lequel le dispositif de mesure (100, 200, 300, 400) est configuré pour effectuer une mesure d'impédance à une pluralité de fréquences, la première fréquence (F1) étant la plus petite fréquence disponible parmi la pluralité de fréquences, la deuxième fréquence (F2) étant la plus grande fréquence disponible parmi la pluralité des fréquences.

13. Méthode de mesure de composition corporelle, la méthode comprenant au moins les étapes successives suivantes :
- mesures d'impédance (1320) sur un utilisateur (U) à au moins deux fréquences différentes (F1, F2, F3), dont une première fréquence (F1) et une deuxième fréquence (F2) plus élevée que la première fréquence (F1),
- comparaison (1330) de l'impédance (Z1) mesurée à la première fréquence (F1) et l'impédance (Z2) mesurée à la deuxième fréquence (F2),
- génération (1340) d'un signal d'erreur sur la base de la comparaison de l'impédance (Z1) mesurée à la première fréquence (F1) et de l'impédance (Z2) mesurée à la deuxième fréquence (F2).

14. Produit programme d'ordinateur comprenant des instructions qui, lorsque le programme est exécuté par un ordinateur, conduisent celui-ci à mettre en oeuvre la méthode de mesure selon la revendication 13.
